(19) Europäisches Patentamt / European Patent Office / Office européen des brevets

(11) **EP 3 216 435 A1**

(12) **EUROPEAN PATENT APPLICATION**

(43) Date of publication:
13.09.2017 Bulletin 2017/37

(51) Int Cl.:
*A61F 13/511* (2006.01)    *A61F 13/537* (2006.01)
*A61F 13/512* (2006.01)

(21) Application number: 16159461.9

(22) Date of filing: 09.03.2016

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 08.03.2016 EP 16159075

(71) Applicant: **The Procter & Gamble Company**
**Cincinnati, OH 45202 (US)**

(72) Inventors:
• **KRAMKOWSKI, Silke**
  **65824 Schwalbach am Taunus (DE)**
• **PONOMARENKO, Ekatarina**
  **65824 Schwalbach am Taunus (DE)**
• **BRUELL, Adele**
  **65824 Schwalbach am Taunus (DE)**

(74) Representative: **Heide, Ute**
**Procter & Gamble Service GmbH**
**IP Department**
**Frankfurter Strasse 145**
**61476 Kronberg im Taunus (DE)**

(54)    **ABSORBENT ARTICLE COMPRISING A TOPSHEET/ACQUISITION LAYER LAMINATE**

(57)    An absorbent article for personal hygiene is provided. The article comprises a longitudinal axis, a transversal axis perpendicular to the longitudinal axis, a liquid permeable topsheet, an acquisition layer, a liquid impermeable backsheet and an absorbent core, wherein the absorbent core is located between the topsheet and backsheet. The topsheet and acquisition layer are jointed to each other to form a topsheet/acquisition layer laminate. The topsheet/acquisition layer laminate comprises three-dimensional protrusions extending from a plane of the topsheet/acquisition layer laminate. The acquisition layer comprises a carded nonwoven fibrous web comprising at least 50%, by weight of the fibrous web, of staple fibers and at least 10%, by weight of the fibrous web, of non-fibrous latex binder, wherein, the staple fibers are autogenously bonded to each other and are bonded to each other by the latex binder.

Fig. 1

**Description**

FIELD OF THE INVENTION

The invention provides an absorbent article for personal hygiene such as a baby diaper, a training pant, a feminine hygiene sanitary napkin or an adult incontinence product. The absorbent article comprises a topsheet/acquisition layer laminate. The acquisition layer of the laminate comprises a carded nonwoven fibrous web with staple fibers and a non-fibrous latex binder, wherein, the staple fibers are autogenously bonded to each other and are bonded to each other by the latex binder.

BACKGROUND OF THE INVENTION

An absorbent article typically comprises a topsheet, a backsheet, and an absorbent core disposed between the topsheet and the backsheet. The absorbent article can further include an acquisition layer and optionally a distribution layer. The acquisition layer is able to receive the liquid bodily exudates from the topsheet to temporary hold them and to distribute them to the materials which are underneath the acquisition layer. For temporarily holding the liquid bodily exudates, the acquisition layer needs to have sufficient void volume.

One of the drawbacks which can be associated with the acquisition layer of absorbent articles is that it may undergo substantial loss in caliper when being subject to an external compressive force. For example, absorbent articles are typically compressed to a relatively high extent while being contained in a closed package for storage and shipment. Moreover, the acquisition layer may undergo compression during manufacturing and during storage of the material in roll-form. However, when the caliper is considerably reduced, the material collapses and the void volume is substantially diminished, thus negatively impacting the performance of the acquisition layer. Therefore, it is highly desirable that the caliper loss of the acquisition layer is reduced and that the caliper recovers well after the compression.

Another important property of an acquisition layer to ensure proper functionality is to have an intimate contact between the acquisition layer and the layer, which is directly above the acquisition layer towards the skin of the wearer. This layer is typically the topsheet. The close contact enables better dewatering of the topsheet by the acquisition layer.

SUMMARY OF THE INVENTION

The invention relates to an absorbent article for personal hygiene comprising a longitudinal axis, a transversal axis perpendicular to the longitudinal axis, a liquid permeable topsheet, an acquisition layer, a liquid impermeable backsheet and an absorbent core, wherein the absorbent core is located between the topsheet and backsheet. The topsheet and acquisition layer are jointed to each other to form a topsheet/acquisition layer laminate comprising the liquid permeable topsheet and the acquisition layer in a face to face relationship. The topsheet/acquisition layer laminate comprises three-dimensional protrusions extending from a plane of the topsheet/acquisition layer laminate. The width of the acquisition layer in a direction parallel to the transversal axis is less than the width of the topsheet in a direction parallel to the transversal axis. The acquisition layer comprises a carded nonwoven fibrous web comprising at least 50%, by weight of the fibrous web, of staple fibers and at least 10%, by weight of the fibrous web, of non-fibrous latex binder, wherein, the staple fibers are autogenously bonded to each other and are bonded to each other by the latex binder.

The carded nonwoven fibrous web may comprise a mixture of at least a first and a second type of staple fibers.

The first type of fibers may be multicomponent fibers, preferably core/sheath bicomponent fibers.

The first type of fibers may have a diameter of from 4 denier to 10 denier.

The second type of fibers maybe monocomponent fibers.

The second type of fibers maybe shaped fibers.

The second type of fibers may have a diameter of from 0.8 denier to 2.5 denier.

The staple fibers of the carded nonwoven fibrous web may comprise at least 20%, by total weight of the staple fibers, of the first type of fibers.

The staple fibers of the carded nonwoven fibrous web may comprise at least 5%, by total weight of the staple fibers, of the second type of fibers.

The carded nonwoven fibrous web may comprise from 65 to 95%, by total weight of the staple fibers, of the first type of fibers and from 5% to 35%, by total weight of the staple fibers, of the second type of fibers.

The carded nonwoven fibrous web may have a basis weight of from 20 gsm to 100 gsm.

The majority or all of three-dimensional protrusions of the topsheet/acquisition layer may protrude towards the absorbent core.

The length of the acquisition layer in a direction parallel to the longitudinal axis may be less than the length of the topsheet in a direction parallel to the longitudinal axis.

BRIEF DESCRIPTION OF THE DRAWINGS

While the specification concludes with claims particularly pointing out and distinctly claiming the present invention, it is believed that the same will be better understood from the following description read in conjunction with the accompanying drawings in which:

Fig. 1 is an absorbent article in the form of a diaper comprising an exemplary topsheet/acquisition layer laminate wherein the length of the acquisition layer is less that the length of the topsheet according to the present invention with some layers partially removed.

Fig. 2 is a transversal cross-section of the diaper of Fig. 1.

Fig. 3 is a transversal cross-section of the diaper of Fig. 1.

Fig. 4 is an absorbent article in the form of a diaper comprising an exemplary topsheet/acquisition layer laminate wherein the three-dimensional protrusions of the topsheet/acquisition layer laminate are only formed where the topsheet overlaps the acquisition layer in the topsheet/acquisition layer laminate, according to the present invention with some layers partially removed.

Fig. 5 is an absorbent article in the form of a diaper comprising an exemplary topsheet/acquisition layer laminate with another type of absorbent core according to the present invention with some layers partially removed.

Fig. 6 is a transversal cross-section of a diaper of Fig. 5.

Fig. 7 is a transversal cross-section of the absorbent article of Fig. 5 taken at the same point as Fig. 6 where channels have formed as a result the absorbent article being loaded with liquid bodily exudates.

Fig. 8 is an absorbent article in the form of a diaper comprising an exemplary topsheet/acquisition layer laminate with an acquisition layer positioned in a front region of the absorbent article according to the present invention with some layers partially removed.

Fig. 9 is an absorbent article in the form of a diaper comprising an exemplary topsheet/acquisition layer laminate with an acquisition layer positioned in a rear region of the absorbent article according to the present invention with some layers partially removed.

Fig. 10A is a perspective view of an apparatus comprising a first and second forming member for forming the topsheet/acquisition layer laminate of the present invention.

Fig. 10B is a perspective view of a portion of the first forming member of the apparatus shown in Fig. 10A.

Fig. 10C is a perspective view of the apparatus shown in Fig. 10A, showing the first forming member intermeshing the second forming member.

Fig. 11A is a perspective view of a three-dimensional protrusion of the topsheet/acquisition layer laminate obtained with the apparatus shown in Fig. 10A.

Fig. 11B is a schematic view of a three-dimensional protrusion of the topsheet/acquisition layer laminate obtained with the apparatus shown in Fig. 10A.

Fig. 11C is a schematic view of a three-dimensional protrusion of the topsheet/acquisition layer laminate obtained with the apparatus shown in Fig. 10A.

Fig. 11D is a schematic view of a three-dimensional protrusion of the topsheet/acquisition layer laminate obtained with the apparatus shown in Fig. 10A.

Fig. 11E is a schematic view of a three-dimensional protrusion of the topsheet/acquisition layer laminate obtained with the apparatus shown in Fig. 10A.

Fig. 11F is a schematic view of a three-dimensional protrusion of the topsheet/acquisition layer laminate obtained with the apparatus shown in Fig. 10A.

Fig. 12A is a schematic view of a three-dimensional protrusion of the topsheet/acquisition layer laminate obtained with the apparatus shown in Fig. 10A.

Fig. 12B is a schematic view of a three-dimensional protrusion of the topsheet/acquisition layer laminate obtained with the apparatus shown in Fig. 10A.

Fig. 12C is a schematic view of a three-dimensional protrusion of the topsheet/acquisition layer laminate obtained with the apparatus shown in Fig. 10A.

Fig. 12D is a schematic view of a three-dimensional protrusion of the topsheet/acquisition layer laminate obtained with the apparatus shown in Fig. 10A.

Fig. 12E is a schematic view of a three-dimensional protrusion of the topsheet/acquisition layer laminate obtained with the apparatus shown in Fig. 10A.

Fig. 13 is a schematic drawing of a through air bonder.

FIG. 14 is a schematic representation of an in plane radial permeability apparatus set up.

FIG. 15 is an alternate view of a portion of the in plane radial permeability apparatus set up shown in FIG. 14.

FIG. 16 is a schematic representation of a fluid delivery reservoir for the in plane radial permeability apparatus set up shown in FIG. 14.

Fig. 17 is a SEM picture of a topsheet/acquisition layer laminate for use in absorbent articles of the present invention (viewing the surface of the acquisition layer)

Fig. 18 is a SEM picture of a topsheet/acquisition layer laminate with an acquisition layer that has only undergone latex bonding, with no autogenous bonds (viewing the surface of the acquisition layer)

Fig. 19 is a SEM picture of a topsheet/acquisition layer laminate with another acquisition layer that has only undergone latex bonding, with no autogenous bonds (viewing the surface of the acquisition layer)

## DETAILED DESCRIPTION OF THE INVENTION

### Definition of terms

As used herein, "absorbent article" refers to devices that absorb and contain body exudates, and, more specifically, refers to devices that are placed against or in proximity to the body of the wearer to absorb and contain the various exudates discharged from the body. Absorbent articles may include diapers (baby diapers and diapers for adult incontinence), pants, inserts, feminine care absorbent articles such as sanitary napkins or pantiliners, and the like. As used herein, the term "exudates" includes, but is not limited to, urine, blood, vaginal discharges, sweat and fecal matter. Preferred absorbent articles of the present invention are disposable absorbent articles, more preferably disposable diapers and disposable pants.

The term "absorbent core" as used herein refers to a component, which is placed or is intended to be placed within an absorbent article and which comprises an absorbent material enclosed in a core wrap. The term "absorbent core" does not include an acquisition or distribution layer or any other component of an absorbent article which is not either an integral part of the core wrap or placed within the core wrap. The absorbent core is typically the component of an absorbent article which comprises all, or at least the majority of, superabsorbent polymer and has the highest absorbent capacity of all the components of the absorbent article.

As used herein, the terms "autogenously bonding", "autogenously bonded" and "autogenous bond" refer to bonding between discrete fibers of the carded nonwoven fibrous web using through-air bonding. Autogenous bonding does not apply solid contact pressure such as is applied for point-bonding or calendaring processes and is done independently of externally added additives which promote or facilitate bonding, such as adhesives, solvents, and the like.

As used herein, "bicomponent" refers to fibers having a cross-section comprising two discrete polymer components, two discrete blends of polymer components, or one discrete polymer component and one discrete blend of polymer components. "Bicomponent fiber" is encompassed within the term "multicomponent fiber." A bicomponent fiber may have an overall cross section divided into two subsections of the differing components of any shape or arrangement, including, for example, concentric core-and-sheath subsections, eccentric core/sheath subsections, side-by-side subsections, radial subsections, etc.

The term "cellulosic fiber" as used herein refers to natural fibers which typically are wood pulp fibers. Applicable wood pulps include chemical pulps, such as Kraft, sulfite, and sulfate pulps, as well as mechanical pulps including, for example, groundwood, thermomechanical pulp and chemically modified thermomechanical pulp. Pulps derived from both deciduous trees (hereinafter, also referred to as "hardwood") and coniferous trees (hereinafter, also referred to as "softwood") may be utilized. The hardwood and softwood fibers can be blended, or alternatively, can be deposited in layers to provide a stratified web.

"Comprise," "comprising," and "comprises" are open ended terms, each specifies the presence of the feature that follows, e.g. a component, but does not preclude the presence of other features, e.g. elements, steps, components known in the art or disclosed herein. These terms based on the verb "comprise" should be read as encompassing the narrower terms "consisting essential of" which excludes any element, step or ingredient not mentioned which materially affect the way the feature performs its function, and the term "consisting of" which excludes any element, step, or ingredient not specified. Any preferred or exemplary embodiments described below are not limiting the scope of the claims, unless specifically indicated to do so. The words "typically", "normally", "advantageously" and the likes also qualify features which are not intended to limit the scope of the claims unless specifically indicated to do so.

As used herein, the term "cross-machine direction" (or CD) is the direction perpendicular to the machine direction.

As used herein, "diaper" and "pant" refers to an absorbent article generally worn by babies, infants and incontinent persons about the lower torso so as to encircle the waist and legs of the wearer and that is specifically adapted to receive and contain urinary and fecal waste. In a pant, as used herein, the longitudinal edges of the first and second waist region are attached to each other to a pre-form waist opening and leg openings. A pant is placed in position on the wearer by inserting the wearer's legs into the leg openings and sliding the pant absorbent article into position about the wearer's lower torso. A pant may be pre-formed by any suitable technique including, but not limited to, joining together portions of the absorbent article using refastenable and/or non-refastenable bonds (e.g., seam, weld, adhesive, cohesive bond, fastener, etc.). A pant maybe preformed anywhere along the circumference of the article (e.g., side fastened, front waist fastened). In a diaper, the waist opening and leg openings are only formed when the diaper is applied onto a wearer by (releasable) attaching the longitudinal edges of the first and second waist region to each other on both sides by a suitable fastening system.

As used herein, "disposable" is used in its ordinary sense to mean an article that is disposed or discarded after a limited number of usage over varying lengths of time, for example, less than 20 usages, less than 10 usages, less than 5 usages, or less than 2 usages. If the disposable absorbent article is a diaper, a pant, sanitary napkin, sanitary pad or wet wipe for personal hygiene use, the disposable absorbent article is most often intended to be disposed after single use.

The term "dry-laid fiber" as used herein means fibers which have been provided in a fluid medium which is gaseous (air).

The term "extensible" as used herein refers to a material, which, upon application of a force, is capable of undergoing an apparent elongation of equal to or greater than at least 100% of its original length in the machine and/or cross-machine directions at or before reaching the breaking force if subjected to the following test:

The MD and CD tensile properties are measured using a method using WSP 110.4 (05) Part B, with a 50 mm sample width, 60 mm gauge length, and 60 mm/min rate of extension.

It may be desirable that a material is capable of undergoing an apparent elongation of equal to or greater than at least 100% or 110% or 120% or 130% up to 200% in the machine and/or cross-machine directions at or before reaching the breaking force according to the Test Method as set out above.

If a material is capable of undergoing an apparent elongation of less than 100 % of its original length if subjected to the above described test, it is "non-extensible" as used herein.

The term "interruptions", as used herein, refers to holes formed in the topsheet and/or acquisition layer during the formation of the topsheet/acquisition layer laminate, and does not include the pores and interstices between fibers typically present in nonwovens.

As used herein, the term "machine direction" (or MD) is the direction parallel to the flow of a material through a manufacturing line.

The term "majority of the three-dimensional protrusions" as used herein means that more than 50%, or more than 60%, or more than 70%, or more than 80%, or more than 90%, or more than 95%, or more than 98% of the three-dimensional protrusions in the topsheet/acquisition layer laminate of the absorbent article.

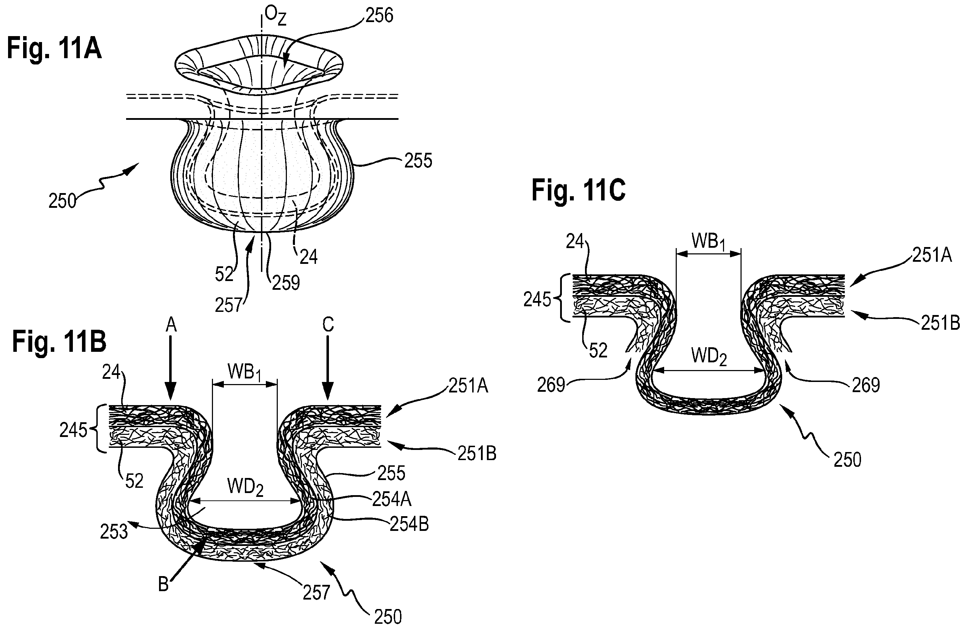

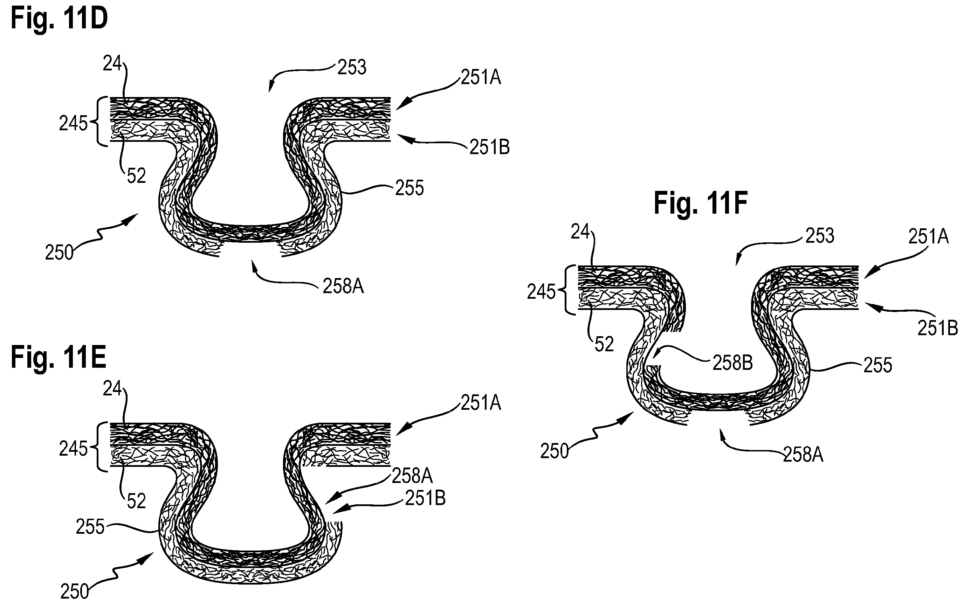

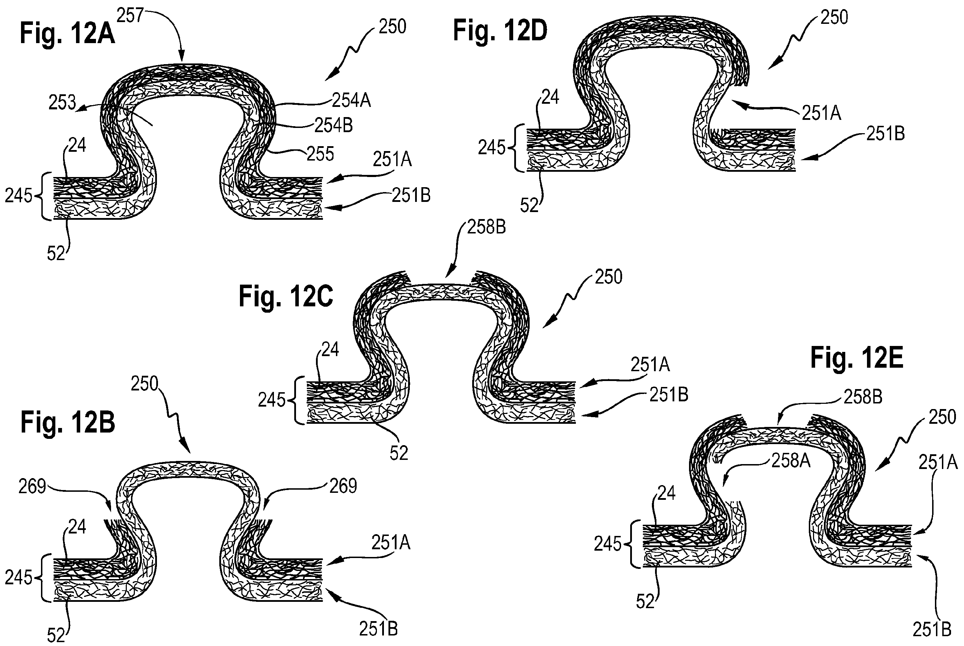

Each of the majority of the three-dimensional protrusions may comprise a base forming an opening, an opposed distal portion and the one or more side wall(s) between the base and the distal portion. A protrusion may have more than one side wall if the protrusion does not take e.g. a circular or ellipsoid shape. For comparison, just like a room typically may have four walls connected to each other at corners instead of having one continuous wall. The base, the distal portion and one or more side wall(s) are formed by fibers such that the three-dimensional protrusion have openings at the base (as exemplary shown in a Figs. 11 and 12). The base, the distal portion and one or more side wall(s) may be formed by fibers of the topsheet and of the acquisition layer when neither the topsheet nor the acquisition layer has interruptions in the area of the three-dimensional protrusions. If, on the other hand, the topsheet and/or the acquisition layer has interruptions, the distal portion and/or one or more of the side wall(s) will have regions made of fibers of only the topsheet or of fibers of only the acquisition layer.

The term "mechanically deforming and combining" as used herein means that the topsheet and acquisition layer are put in a face to face relationship and can be simultaneously mechanically deformed between a first and second roll and intimately combined at the same time. The mechanical deformation of the topsheet and acquisition layer depends on the process, the required apparatus but also on the properties of the topsheet and acquisition layer, i.e. apparent elongation of the fibers, fiber mobility, ability to deform and stretch in the area where the three-dimensional protrusions of the topsheet/acquisition layer laminate are formed, ability to undergo plastic deformation which sets after existing the first and second roll, or springing partially back due to elastic recovery. Due to the difference between the topsheet and the acquisition layer material with respect to these properties, interruptions may or may not form in the topsheet and/or the acquisition layer upon mechanical deformation and combination.

The mechanical deformation may comprise engaging the topsheet and the acquisition layer together between a first and second forming member such that a plurality of deformations comprising three-dimensional protrusions are obtained. The three-dimensional protrusions are formed from the fibers of the topsheet and the acquisition layer. A majority of the three-dimensional protrusions is defined by a base forming an opening, an opposed distal portion and one or more side walls between the base and the distal portion. The base, the distal portion and the one or more side walls are formed by fibers such that the majority of the three-dimensional protrusions have openings at the base, as shown e.g. in Figs. 11A-F and Figs. 12A-E.

As used herein, "monocomponent" refers to fiber formed of a single polymer component or single blend of polymer components, as distinguished from bicomponent or multicomponent fiber.

As used herein, "multicomponent" refers to fiber having a cross-section comprising two or more discrete polymer components, two or more discrete blends of polymer components, or at least one discrete polymer component and at least one discrete blend of polymer components.

"Multicomponent fiber" includes, but is not limited to, "bicomponent fiber."

As used herein, the term "non-consolidated fibers" refers to fibers which are not formed into a self-sustaining, integral web.

The term "nonwoven web" as used herein refers to a manufactured material, web, sheet or batt of directionally or randomly oriented fibers, bonded by friction, and/or cohesion and/or adhesion, excluding paper and products which are woven, knitted, tufted, stitch-bonded, incorporating binding yarns or filaments, or felted by wet milling, whether or not additionally needled. The fibers may be of natural or man-made origin. The fibers may be staple or continuous filaments or be formed in situ. The porous, fibrous structure of a nonwoven may be configured to be liquid permeable or impermeable, as desired.

As used herein, a "pantiliner" and a "sanitary napkin" generally have two end regions and a middle region (i.e. a crotch region). The pantiliner and the sanitary napkin has a body-facing surface and a garment facing surface. The size and shape of the absorbent structure positioned between the topsheet and the backsheet can be altered to meet absorbent capacity requirements, and to provide comfort to the wearer. The garment facing surface of the pantiliner and of the sanitary napkin can have thereon pressure sensitive adhesive for affixing to a wearer's undergarments. Typically, such adhesive is covered with a release strip which is removed before affixing to the undergarment. Pantiliners can also be provided with lateral extensions known commonly in the art as "flaps" or "wings" intended to extend over and cover the panty elastics in the crotch region of the user's undergarment. However, wings are normally not used with pantiliners but are more often used in sanitary napkins. Sanitary napkins and pantiliners of the present invention comprise barrier leg cuffs.

The term "substantially free of absorbent material" or "substantially absorbent material free" as used herein means that the basis weight of the absorbent material in the substantially absorbent material free areas is at least less than 10%, in particular less than 5%, or less than 2%, of the basis weight of the absorbent material in the rest of the absorbent core.

The term "superabsorbent polymers" (herein abbreviated as "SAP") as used herein refer to absorbent materials which are cross-linked polymeric materials that can absorb at least 10 times their weight of an aqueous 0.9% saline solution as measured using the Centrifuge Retention Capacity (CRC) test (EDANA method WSP 241.2-05E). The SAP of the invention may in particular have a CRC value of more than 20 g/g, or more than 25 g/g, or from 20 to 50 g/g, or from 20 to 40 g/g, or 25 to 35 g/g. The SAP useful in the invention includes a variety of water-insoluble, but water-swellable polymers capable of absorbing large quantities of liquid bodily exudates.

The term "topsheet/acquisition layer laminate" as used herein refers to an intimate combination of a topsheet with an acquisition layer, both disposed in a face to face relationship. The topsheet has a first and second surface. The first surface of the topsheet is facing towards the body of the wearer when the absorbent article is in use. The acquisition layer has a first surface and a second surface. The second surface of the acquisition layer is facing towards the backsheet. The topsheet and the acquisition layer can have undergone a simultaneous and joint mechanical deformation while the topsheet and the acquisition layer are combined with each other. The topsheet/acquisition layer laminate comprises deformations forming three-dimensional protrusions and extending out of the plane of the laminate.

In the topsheet/acquisition layer laminate, the topsheet and acquisition layer may be in an intimate contact with each other. The topsheet/acquisition layer laminate may be formed by nesting together the topsheet and acquisition layer, whereby the three-dimensional protrusions of the topsheet coincide with and fit together with the three-dimensional protrusions of the acquisition layer, as shown in Figs. 11 and 12.

Neither the topsheet nor the acquisition layer of the topsheet/acquisition layer laminate which are nested together may have interruptions (see e.g. Figs. 11A, 11B and 12A).

Alternatively or in addition, the topsheet/acquisition layer laminate, wherein the topsheet and acquisition layer are nested together, may have interruptions in the area of at least some, or of all, of the three-dimensional protrusions in the topsheet, in the acquisition layer, or in both. If the topsheet and acquisition layer both comprise interruptions in the area of the three-dimensional protrusions, the interruptions in the topsheet in the area of the three-dimensional protrusions of the topsheet/acquisition layer laminate will not coincide with the interruptions in the acquisition layer in the area of the three-dimensional protrusions of the topsheet/acquisition layer laminate. Examples of topsheet/acquisition layer laminates having interruptions in interruptions in the area the three-dimensional protrusions in the topsheet, in the acquisition layer, or in both, are shown in Figs. 11B to 11F and in Figs. 12B to 12E.

The term "web" as used herein means a material capable of being wound into a roll. Webs may be nonwovens.

The term "wet-laid fiber" as used herein comprises cellulosic fibers which have been suspended in an aqueous medium, such as water, before being converted into a web and dried according to a wet-laid papermaking process.

**General description of the absorbent article 20**

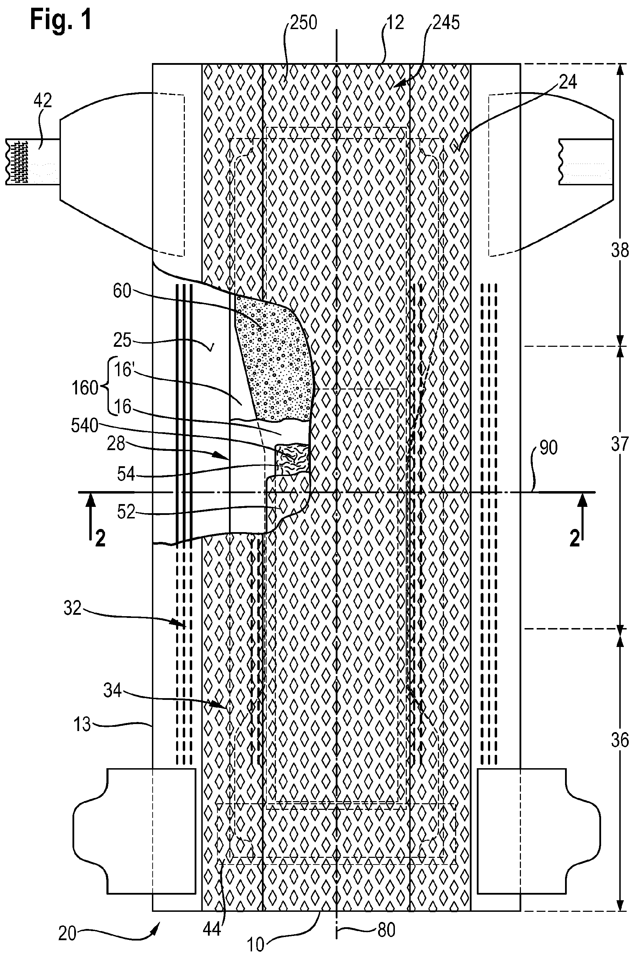

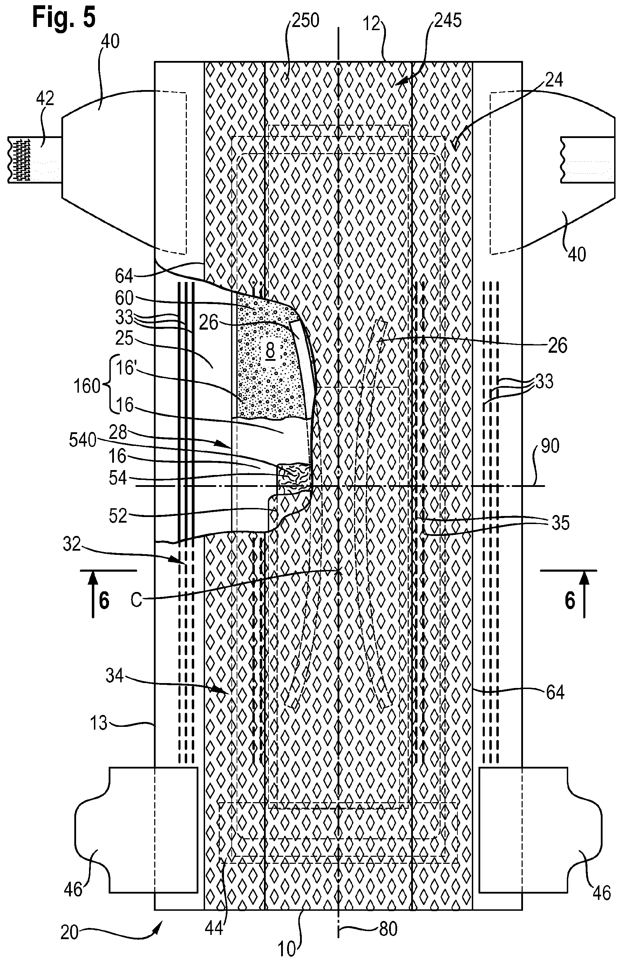

An exemplary absorbent article 20 in which the absorbent core 28 of the invention can be used is a taped diaper 20 as represented in Fig. 1, Fig. 4 and Fig. 5. Fig. 1, Fig. 4 and Fig. 5 are top plan views of the exemplary diaper 20, in a flat-out state, with portions of the structure being cut-away to more clearly show the construction of the diaper 20. This diaper 20 is shown for illustration purpose only as the invention may be used for making a wide variety of diapers or other absorbent articles.

The absorbent article 20 comprises a topsheet/acquisition layer laminate 245 formed from a liquid permeable topsheet 24 and an acquisition layer 52. In other words, the absorbent article 20 comprises a liquid permeable topsheet 24 and an acquisition layer 52 wherein the topsheet 24 and acquisition layer 52 are joined to form a topsheet/acquisition layer laminate 245.

The absorbent article 20 comprises a liquid impermeable backsheet 25 and an absorbent core 28 between the topsheet 24 and the backsheet 25. The absorbent article 20 comprises a front edge 10, a back edge 12, and two longitudinal side edges 13. The front edge 10 is the edge of the absorbent article 20 which is intended to be placed towards the front of the user when worn, and the back edge 12 is the opposite edge. The absorbent article 20 may be notionally divided by a longitudinal axis 80 extending from the front edge 10 to the back edge 12 of the absorbent article 20 and dividing the absorbent article 20 in two substantially symmetrical halves relative to this axis, when viewing the absorbent article 20 from the wearer facing side in a flat out configuration, as exemplarily shown in Fig. 1, Fig. 4 and Fig. 5.

The absorbent article 20 may be divided by a lateral axis 90 into a front half and a back half of equal length measured along the longitudinal axis 80, when the absorbent article 20 is in a flat, laid-out state. The absorbent article's lateral axis 90 is perpendicular to the longitudinal axis 80 and is placed at half the longitudinal length of the absorbent article 20.

The longitudinal dimension of the absorbent article extends substantially parallel to the longitudinal axis 80 and the lateral dimension extends substantially parallel to the lateral axis 90.

The absorbent article 20 may be notionally divided into a front region 36, a back region 38 and a crotch region 37 located between the front region 36 and the back region 38 of the absorbent article 20. Each of the front, back and crotch regions are 1/3 of the longitudinal dimension of the absorbent article 20.

The absorbent article may comprise further optional other features such as leg cuffs 32 and/or barrier cuffs 34, front and/or back waist features such as front and/or elastic waistbands attached adjacent to the respective front and/or back waist edge of the absorbent article.

The topsheet 24, the backsheet 26, and the absorbent core 28 may be assembled in a variety of well known configurations, in particular by gluing or heat embossing. Exemplary diaper configurations are described generally in US3,860,003; US5,221,274; US5,554,145; US5,569,234; US5,580,411; and US6,004,306.

The diaper 20 may comprise leg cuffs 32 which provide improved containment of liquids and other body exudates. Leg cuffs 32 may also be referred to as leg bands, side flaps, barrier cuffs, or elastic cuffs. Usually each leg cuffs will comprise one or more elastic string 33, represented in exaggerated form on Fig. 1, comprised in the diaper for example between the topsheet and backsheet in the area of the leg openings to provide an effective seal while the diaper is in use. It is also usual for the leg cuffs to comprise "stand-up" elasticized flaps (barrier leg cuffs 34) which improve the containment of the leg regions.

The absorbent article 20 may comprise a distribution layer 54 provided beneath the topsheet/acquisition layer laminate towards the backsheet, such as between the topsheet/acquisition layer laminate and the absorbent core.

The function of a distribution layer 54 is to spread the insulting fluid liquid over a larger surface within the article so that the absorbent capacity of the absorbent core can be more efficiently used. Distribution layers maybe made of a nonwoven material based on synthetic or cellulosic fibers and having a relatively low density. The distribution layer may typically have an average basis weight of from 30 g/m$^2$ to 400 g/m$^2$, in particular from 80 g/m$^2$ to 300 g/m$^2$.

The distribution layer may for example comprise intra-fiber cross-linked cellulose fibers. The intra-fiber cross-linked cellulosic fibers may be crimped, twisted, or curled, or a combination thereof including crimped, twisted, and curled. The intra-fiber cross-linked cellulosic fibers provide higher resilience and therefore higher resistance to the first absorbent layer against the compression in the product packaging or in use conditions, e.g. under the weight of a wearer. This provides a relatively high void volume, permeability and liquid absorption, and hence reduced leakage and improved dryness.

The distribution layer comprising intra-fiber cross-linked cellulose fibers, may comprise other fibers, but this layer may advantageously comprise at least 50%, or 60%, or 70%, or 80%, or 90% or even up to 100%, by weight of the layer, of intra-fiber cross-linked cellulose fibers. Examples of such mixed layers of intra-fiber cross-linked cellulose fibers with other fibers may comprise 60% to 80%, or 60% to 75% by weight of intra-fiber cross-linked cellulose fibers, 5% to 20%, or 5% to 15% by weight of polyester (PET) fibers, and 5 % to 20%, or 5% to 15% by weight of untreated pulp fibers. In another example, the distribution layer may comprise 65 % to 80% by weight of intra-fiber cross-linked cellulose fibers, 10 % to 20% by weight of lyocell fibers, and 5% to 15% by weight of PET fibers. In another example, the distribution layer may comprise 68 % by weight of intra-fiber cross-linked cellulose fibers, 16% by weight untreated pulp fibers, and 16 % by weight PET fibers.

A further layer may be used in addition to a acquisition layer and the distribution layer. For example a tissue layer may be placed between the first acquisition layer and the distribution layer and/or between the distribution layer and the absorbent core. The tissue may have enhanced capillarity distribution properties compared to the acquisition layer. The tissue and the first acquisition layer may be of the same size or may be of different size, for example the tissue layer may extend further in the back of the absorbent article than the first acquisition layer. An example of hydrophilic tissue is a 8 - 20 g/m$^2$ tissue made of cellulose fibers, which maybe supplied e.g. by supplier Havix.

The length of the absorbent article 20 can be measured along the longitudinal axis 80 from the front edge 10 to the back edge 12 of the absorbent article 20. The topsheet 24, acquisition layer 52, distribution layer 54 and absorbent core 28 each have a width which can be measured from their respective transversal edges and in parallel to the transversal axis 90.

The acquisition layer 52 in the topsheet/acquisition layer laminate 245 may be positioned in the front region 36, in the crotch region 37 and/or in the back region of the absorbent article 20.

The absorbent core 28 of the present invention may comprise as absorbent material 60 a blend of cellulosic fibers (so called "airfelt") and superabsorbent polymers in particulate form encapsulated in one or more substrates, see for example US 5,151,092 (Buell). Alternatively, the absorbent core 28 may be airfelt free as described in detail below.

### "Airfelt-free" absorbent core 28

The absorbent core 28 of the invention may comprise an absorbent material 60 enclosed within a core wrap 160. The absorbent material 60 may comprise from 80% to 100% of superabsorbent polymer (SAP), such as SAP particles, by total weight of the absorbent material 60. The core wrap 160 is not considered as an absorbent material 60 for the purpose of assessing the percentage of SAP in the absorbent core 28.

By "absorbent material" it is meant a material which has absorbency and/or liquid retaining properties, such as SAP, cellulosic fibers as well as some hydrophilically treated synthetic fibers. Typically, adhesives used in making absorbent cores have no absorbency properties and are not considered as absorbent material. The SAP content may be substantially higher than 80%, for example at least 85%, at least 90%, at least 95% and even up to and including 100% of the weight of the absorbent material 60 contained within the core wrap 160. This above SAP content substantially higher than 80% SAP may provide a relatively thin absorbent core 28 compared to conventional absorbent cores typically comprising between 40-60% SAP and 40-60% of cellulosic fibers. The absorbent material 60 of the invention may in particular comprise less than 10% weight percent, or less than 5% weight percent, or even be substantially free of natural and/or synthetic fibers. The absorbent material 60 may advantageously comprise little or no cellulosic fibers, in particular the absorbent core 28 may comprise less than 15%, 10%, or 5% (airfelt) cellulosic fibers by weight of the absorbent core 28, or even be substantially free of cellulose fibers. Such absorbent core 28 may be relatively thin and thinner than conventional airfelt cores. Fig. 1, Fig. 2 and Fig. 3 are illustrations of an absorbent article 20 comprising an "airfelt-free" absorbent core 28.

"Airfelt-free" absorbent cores 28 comprising relatively high amount of SAP with various absorbent core designs have been proposed in the past, see for example in US 5,599,335 (Goldman), EP1447066A1 (Busam), WO95/11652 (Tanzer), US2008/0312622A1 (Hundorf), and WO2012/052172 (Van Malderen).

The absorbent core 28 of the invention may comprise adhesive for example to help immobilizing the SAP within the core wrap 160 and/or to ensure integrity of the core wrap, 160 in particular when the core wrap 160 is made of one or more substrates. The core wrap 160 will typically extend over a larger area than strictly needed for containing the absorbent material 60 within.

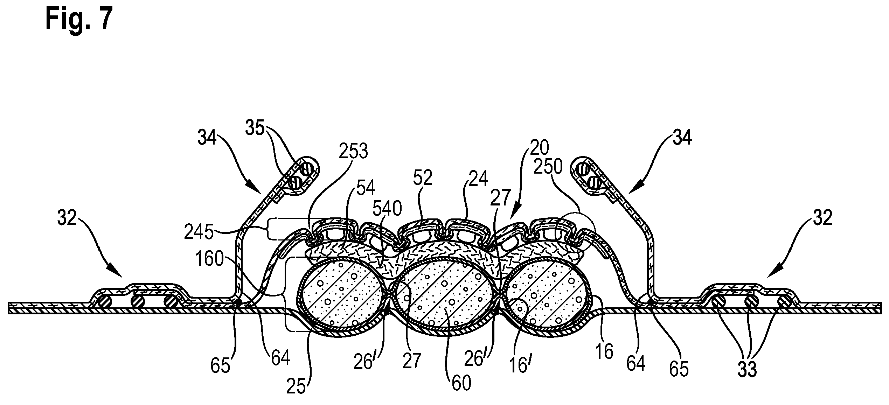

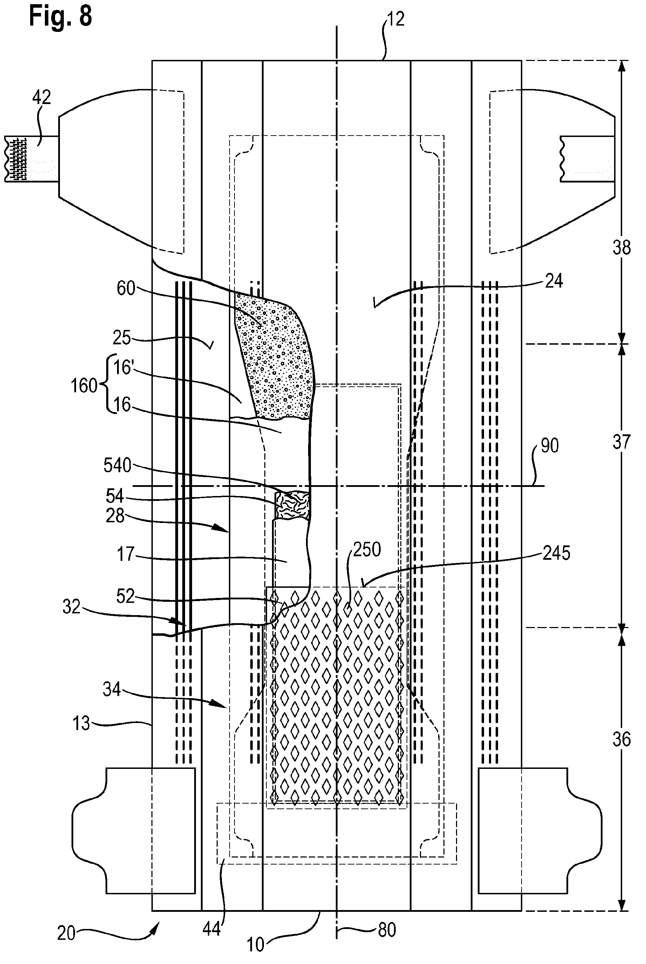

The absorbent core 28 may comprise areas which are free of absorbent materials. Such areas may take the shape of elongate areas extending substantially along the longitudinal dimension of the absorbent article. For example, the absorbent core may comprise a pair of elongate absorbent material free areas, one being arranged on either side along the longitudinal axis of the absorbent article. The elongate absorbent material free areas may be provided at least in the crotch region of the absorbent article. An example of such an absorbent article is shown in Fig. 7 (dry state of the article) and Fig. 8. Fig. 7 illustrates the dry state of the article while Fig. 8 depicts an absorbent core after it has absorbed liquid such that the absorbent material, such as SAP, is swollen to multiple times its initial volume, whereby channels are formed in the absorbent material free areas. The absorbent material free areas may have a longitudinal extension of 1 to 30 cm, or of 2 to 20 cm, or of 5 to 20 cm. The width of the absorbent material may be less than 2 cm, or less than 1 cm. The ratio of length to width may be between 40:1 and 5:1, or between 30:1 to 8:1.

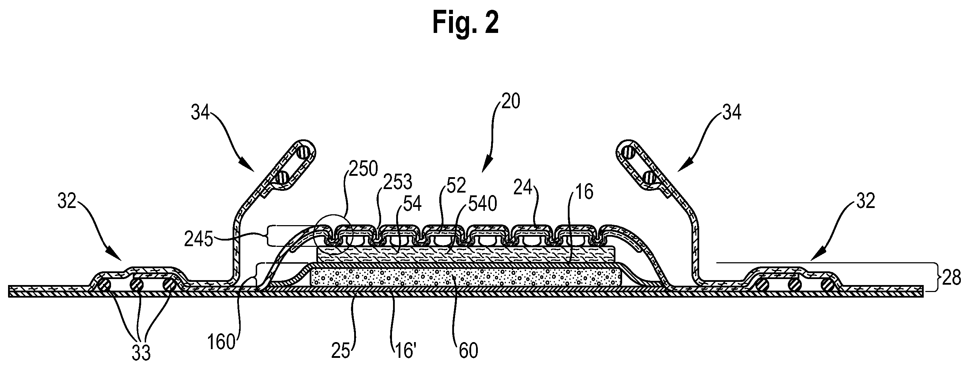

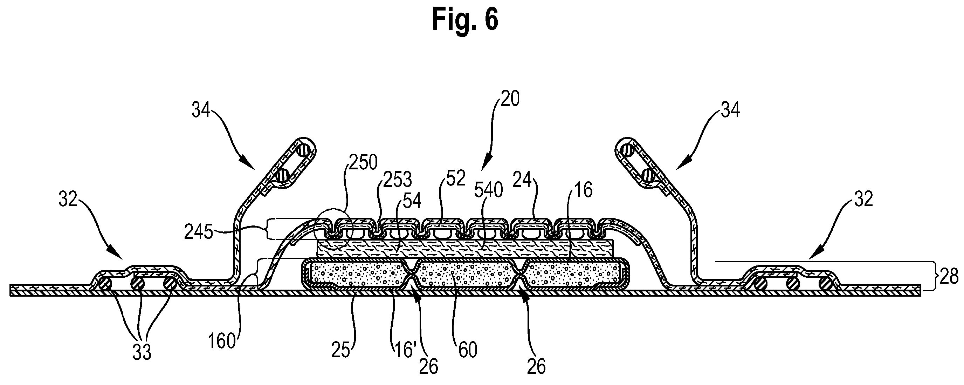

The absorbent material 60 may be encapsulated in one or more substrates. The core wrap 160 comprises a top side 16 facing the topsheet 24 and a bottom side 16' facing the backsheet 25. The core wrap 160 may be made of a single substrate folded around the absorbent material 60. The core wrap 160 may be made of two substrates (one mainly providing the top side 16 and the other mainly providing the bottom side 16') which are attached to another, as exemplarily shown in Fig. 2. Typical configurations are the so-called C-wrap and/or sandwich wrap. In a C-wrap, as exemplarily shown in Fig. 6, the longitudinal and/or transversal edges of one of the substrate are folded over the other substrate to form flaps. These flaps are then bonded to the external surface of the other substrate, typically by bonding with an adhesive. The so called C-wrap construction can provide benefits such as improved resistance to bursting in a wet loaded state compared to a sandwich seal.

The core wrap 160 may be formed by any materials suitable for receiving and containing the absorbent material 60. The core wrap 160 may in particular be formed by a nonwoven web, such as a carded nonwoven, spunbond nonwoven ("S") or meltblown nonwoven ("M"), and laminates of any of these. For example spunmelt polypropylene nonwovens are suitable, in particular those having a laminate web SMS, or SMMS, or SSMMS, structure, and having a basis weight range of about 5 gsm to 15 gsm. Suitable materials are for example disclosed in US 7,744,576, US2011/0268932A1, US2011/0319848A1 or US2011/0250413A1. Nonwoven materials provided from synthetic fibers may be used, such as polyethylene (PE), polyethylene terephthalate (PET) and in particular polypropylene (PP).

**General structure and properties of the topsheet/acquisition layer laminate 245**

A topsheet/acquisition layer laminate 245 having a three-dimensional structure is provided. The topsheet/acquisition layer laminate 245 has a first and a second surface, wherein the second surface faces towards the body of the wearer when the article is in use and the first surface faces towards the backsheet 25 and.

The acquisition layer has a first and a second surface and the topsheet has a first and a second surface.

The topsheet 24 and the acquisition layer 52 are aligned in a face to face relationship such that the second surface of the topsheet 24 is in contact with the first surface of the acquisition layer 52. The first surface of the topsheet 24 faces towards the body of the wearer and the second surface of the acquisition layer 52 faces towards the backsheet 25 of the absorbent article 20 when the absorbent article 20 is in use.

The first surface of the topsheet/acquisition layer laminate 245 comprises the second surface of the acquisition layer 52 and a portion of the second surface of the topsheet (given that the width of the acquisition layer is smaller than the width of the topsheet, the side portions of the topsheet along the longitudinal dimension will form the first surface of the topsheet/acquisition layer laminate). The second surface of the topsheet/acquisition layer laminate is formed by the first surface of the topsheet.

The topsheet/acquisition layer laminate 245 comprises mechanical deformations forming three-dimensional protrusions 250 extending from a plane of the topsheet/acquisition layer laminate 245. The topsheet 24 and acquisition layer 52 may be in an intimate contact with each other.

According to a process detailed below, the topsheet 24 and the acquisition layer 52 can be simultaneously mechanically deformed and combined together in a face to face relationship such as to provide a topsheet/acquisition layer laminate 245. This means that both topsheet 24 and acquisition layer 52 can be mechanically deformed and combined together at the same time during the process.

The three-dimensional protrusions 250 are formed from the fibers of the topsheet 24 and the acquisition layer 52. A majority of the three-dimensional protrusions 250 each comprises a base 256 forming an opening and having a measured protrusion base width according to the Protrusion Base Width Test Method, an opposed distal portion 257, and one or more side walls 255 between the bases 256 and the distal portions 257 of the majority of the three-dimensional protrusions 250. The base 256, distal portion 257 and the one or more side walls 255 are formed by fibers such that the majority of the three-dimensional protrusions 250 have openings at the base 256, as shown in Figs. 11A-F and Fig. 12A-E. At least 50%, or at least 80%, or at least 90%, or at least 95% of the three-dimensional protrusions 250 of the topsheet/acquisition layer laminate 245 may only have openings at the base. The majority of the three-dimensional protrusions may be obtained by the mechanical process described in detail below.

The fibers may substantially or completely surround the one or more side wall(s) 255 of the majority of the three-dimensional protrusions 250. This means that there are multiple fibers which contribute to form a portion of the side walls 255 and distal portion 257 of a three-dimensional protrusion 250. The phrase "substantially surround" does not require that each individual fiber be wrapped substantially or completely around the side walls 255 of the majority of the three-dimensional protrusions 250.

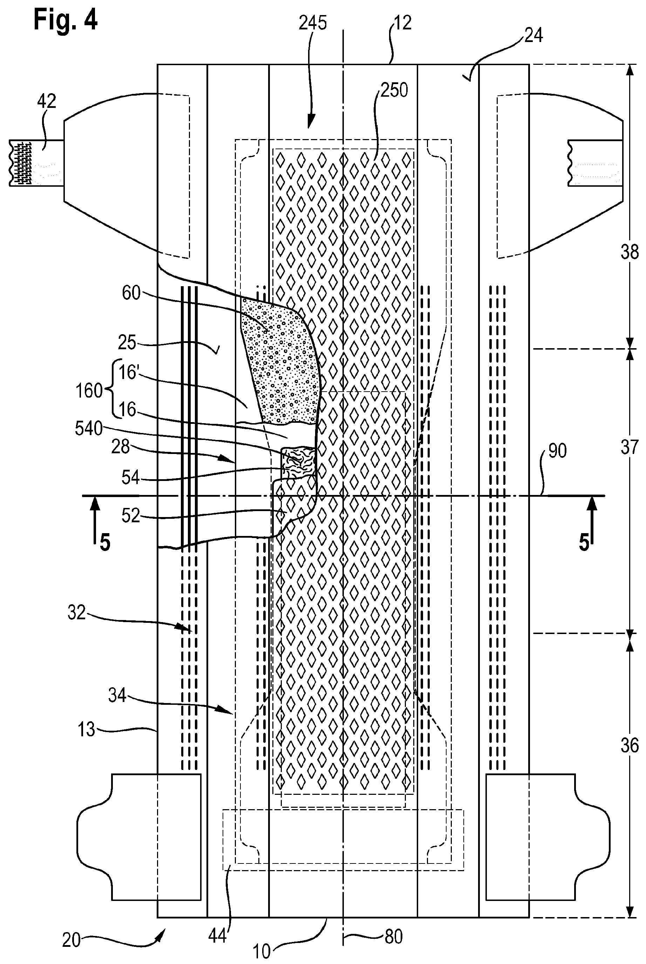

The majority of the three-dimensional protrusions 250 of the topsheet/acquisition layer laminate 245 is at least be present in the area where the topsheet 24 overlaps the acquisition layer 52 in the topsheet/acquisition layer laminate 245. The three dimensional protrusions may only be present in the area where the topsheet 24 overlaps the acquisition layer 52 (shown e.g. in Fig. 4). Alternatively, three-dimensional protrusions may also be present in the area of the topsheet/acquisition layer laminate which does not comprise the acquisition layer (i.e. in the area where the topsheet extends outward beyond the acquisition layer).

The absorbent article may comprise a pair gasketing cuffs 32 provided adjacent to the longitudinal side edges 13 of the absorbent article. The gasketing cuffs have a free end, which extends outward from the topsheet towards the body of the wearer when the article is in use. The gasketing cuffs further have a fixed end, where they are attached to the absorbent article. In such absorbent articles, the acquisition layer may not extend across the complete lateral dimension between the two gasketing cuffs but may, instead, be smaller in width. Then, the three-dimensional protrusions (in addition to being present in the area where the topsheet overlaps the acquisition layer) may be present in the area where the topsheet extends beyond the acquisition layer in the lateral dimension (i.e. parallel to the lateral axis) but may not be present in the area where the fixed ends of gasketing cuffs are attached to the absorbent article and may also not be present in the area laterally outward beyond the gasketing cuffs towards the longitudinal side edges 13 of the absorbent article (shown e.g. in Fig. 1).

The three-dimensional protrusions 250 of the topsheet/acquisition layer laminate 245 may have a measured protrusion height of at least 0.3 mm according to the Protrusions Height Test Method as described below.

The three-dimensional protrusions 250 of the topsheet/acquisition layer laminate 245 may have a measured protrusion height from 0.3 mm to 5 mm or from 0.7 mm to 3 mm or from 1.0 mm to 2.0 mm according to the Protrusions Height Test Method as described below.

The majority of the three-dimensional protrusions 250 of the topsheet/acquisition layer laminate 245 may have a measured protrusion base width of the three-dimensional protrusions 250 of at least 0.5 mm according to the Protrusions Base Width Test Method as described below.

The three-dimensional protrusions 250 of the topsheet/acquisition layer laminate 245 may have a measured protrusion base width of the three-dimensional protrusions 250 from 0.5 mm to 8 mm or from 0.5 mm to 5 mm or from 0.5 mm to 3.0 mm or from 1.0 mm to 2.5 mm or from 1.5 mm to 2.5 mm according to the Protrusions Base Width Test Method as described below.

The topsheet 24 and the acquisition layer 52 in the topsheet/acquisition layer laminate 250 may be in a substantially closer contact with each other when compared to a topsheet 24 simply laid down on top of an acquisition layer 52 without any subsequent joint mechanical deformation.

In addition, the topsheet/acquisition layer laminate 245 comprises three-dimensional protrusions 250. As set out above, the majority of the three-dimensional protrusions 250 have a certain minimum measured protrusion height and a measured protrusion base width according to the Protrusion Height Test Method and Protrusions Base Width Test Method as described below. The majority of the three-dimensional protrusions 250 provide therefore additional void volume inside the protrusions in addition to the void volume provide by the materials of the topsheet and, especially, of the acquisition layer itself (e.g. in the interstices between the fibers of the respective materials) in order to acquire the liquid bodily exudates. Hence, the liquid bodily exudates can be transmitted more efficiently from the topsheet/acquisition layer laminate 245 to the optional distribution layer 54 and to the absorbent core, which improves the dryness of the topsheet 24 of the topsheet/acquisition layer laminate 245. A loss in Protrusion Height relates to a loss in void volume of the topsheet/acquisition layer laminate. Hence, a reduced loss in Protrusion Height after compression of the material provides an improved material versus a topsheet/acquisition layer laminate having higher loss in Protrusion Height.

The width of the acquisition layer 52 in a direction parallel to the transversal axis 90 is less than a width of the topsheet 24 in a direction parallel to the transversal axis 90 of the absorbent article 20. If the width of both topsheet 24 and acquisition layer 52 were the same, wicking of the liquid bodily exudates underneath the gasketing cuffs 32 might occur. Hence, the liquid bodily exudates might not be properly absorbed by the absorbent core 28, which may lead to leakage of the liquid bodily exudates out of the absorbent article. If the width of the acquisition layer 52 in a direction parallel to the transversal axis 90 is less that the width of the topsheet 24 in a direction parallel to the transversal axis 90, the acquisition layer 52 which may receive the liquid bodily exudates from the topsheet 24 can directly transmit the liquid bodily exudates to the optional distribution layer 54 or to the absorbent core in order to be subsequently absorb by the absorbent core 28. Hence, the liquid bodily exudates temporary stored in the acquisition layer 52 of the topsheet/acquisition layer laminate 245 will not readily be drawn towards and underneath the gasketing cuffs 32 by capillary forces. This arrangement can help to reduce leakage of liquid out of the absorbent article.

The width of the acquisition layer 52 in a direction parallel to the transversal axis 90 of the topsheet/acquisition layer laminate 245 may not be more than 40% wider than the width of the optional distribution layer 54 and/or more than 20% wider than the width of the absorbent core 28 in a direction parallel to the transversal axis 90. In that case, the liquid bodily exudates may not accumulate at or adjacent to the transversal edges of the acquisition layer.

A portion of the backsheet 25 may be joined to the topsheet 24 at or adjacent to the longitudinal edges of the first surface of the topsheet/acquisition layer laminate 245. The longitudinal edges of the first surface of the topsheet/acquisition layer laminate 245 do not comprise any acquisition layer 52.

A carrier layer may be disposed between the topsheet/acquisition layer laminate 245 and the dry-laid fibrous structure. According to the method used for making the three-dimensional structure of the topsheet/acquisition layer laminate 245, when the topsheet 24 and acquisition layer 52 are mechanically deformed together, they are typically subjected to certain mechanical stresses. As a consequence, holes might unintentionally occur due to the mechanical strain applied to the materials in a few of the three-dimensional protrusions or even in the land areas. When the optional distribution layer 54, or the absorbent core comprises dry-laid fibers, the fibers 540 may pass through the unintentional holes formed at the topsheet/acquisition layer laminate 245 and contact undesirably the skin of the wearer. The carrier layer may act as a barrier layer to impede the fibers 540 from passing through the holes of the topsheet/acquisition layer laminate 245. Also, the carrier layer may help the transfer of the liquid bodily exudates from the topsheet/acquisition layer laminate 245 to the dry-laid fibrous structure.

Alternatively, the carrier layer may be disposed between the distribution layer and the absorbent core 28. Hence, the carrier layer may help to distribute and transfer of the liquid bodily exudates from the distribution layer 54 to the absorbent core 28.

The length of the acquisition layer 52 in the topsheet/acquisition layer laminate 245 in a direction parallel to the longitudinal axis may be less than the length of the topsheet 24 of the absorbent article 20, in a direction parallel to the longitudinal axis, as shown in Fig. 4. When the length of the acquisition layer 52 in the topsheet/acquisition layer laminate 245 is less than the length of the topsheet 24, the liquid bodily exudates may not be readily drawn towards the longitudinal edges 10, 12 of the absorbent article 20, which can help to reduce leakage.

The length of the acquisition layer 52 in the topsheet/acquisition layer laminate 245 may be less than the length of the absorbent core 28 taken along the longitudinal axis 80 of the absorbent article 20, see for example Fig. 4.

The acquisition layer 52 of the topsheet/acquisition layer laminate 245 may be positioned in the front region 36 and in the crotch region 37 of the absorbent article 20 but not in the back region 38, as shown in Fig. 8. In that case, positioning the acquisition layer 52 of the topsheet/acquisition layer laminate 245 in the front region 36 of the absorbent article 20 can help to acquire and distribute the liquid bodily exudates such as urine, in the area of the pee point where liquid is typically introduced into the absorbent article during use.

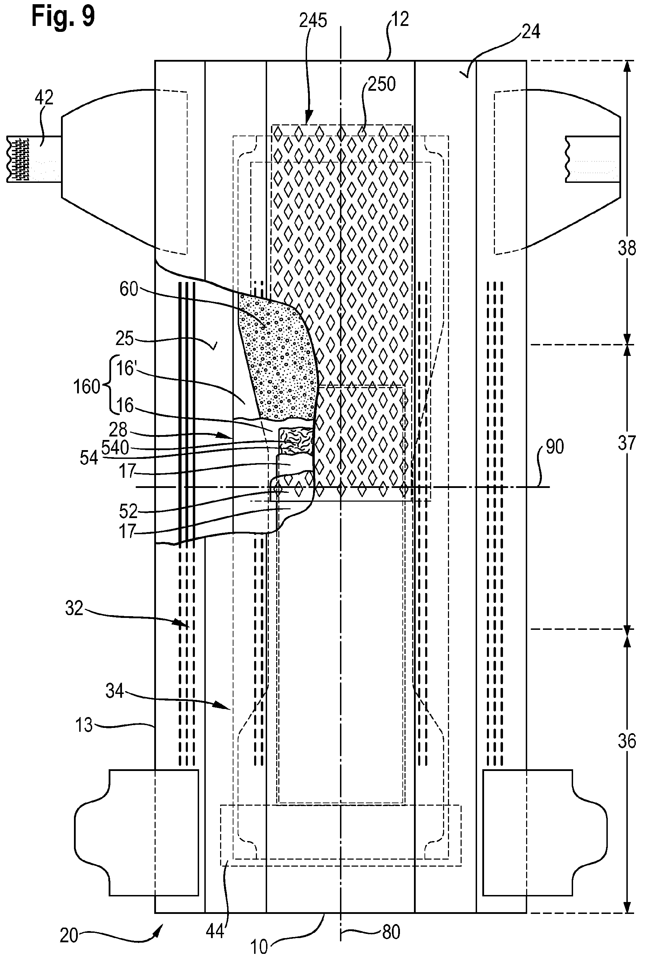

The acquisition layer 52 of the topsheet/acquisition layer laminate 245 may be positioned in the back region 38 and in the crotch region 37, but not in the front region 36, of the absorbent article 20, as shown in Fig. 9. Positioning the acquisition layer 52 of the topsheet/acquisition layer laminate 245 in the back region 38 of the absorbent article 20 helps acquiring feces, especially when the feces have a low viscosity.

Alternatively, the acquisition layer 52 of the topsheet/acquisition layer laminate 245 may be portioned in the front, crotch and back region 36, 37, 38 of the absorbent article.

The majority of the three-dimensional protrusions 250 of the topsheet/acquisition layer 245 may protrude towards the absorbent core 28 or, though less desirable, may protrude towards the body of the wearer when the absorbent article is in use.

The majority of the three-dimensional protrusions 250 of the topsheet/acquisition layer laminate 245 may be distributed along a surface corresponding to at least 50 % to 80%, or at least 50% to 95% of the entire surface of the topsheet/acquisition layer laminate.

The majority of the three-dimensional protrusions 250 of the topsheet/acquisition layer laminate 245 may be distributed along an area corresponding to at least 50% of the area where the topsheet 24 and the acquisition layer 52 overlap each other.

In the surface where the three-dimensional protrusions 250 of the topsheet/acquisition layer laminate 245 may be provided, they may be uniformly distributed.

An area of 10 cm$^2$ of the topsheet/acquisition layer laminate 245 may comprise from 5 to 100 three-dimensional protrusions 250 from 10 to 50 three-dimensional protrusions 250 or from 20 to 40 three-dimensional protrusions 250.

The majority of the three-dimensional protrusions 250 may be disposed in any suitable arrangement across the plane of the topsheet/acquisition layer laminate 245. Suitable arrangements include, but are not limited to: staggered arrangements, and zones. In some cases, the topsheet/acquisition layer laminate 245 may comprise both three-dimensional protrusions 250 and other features known in the art such as embossments and apertures.

**Mechanical deformations and resulting three-dimensional protrusions**

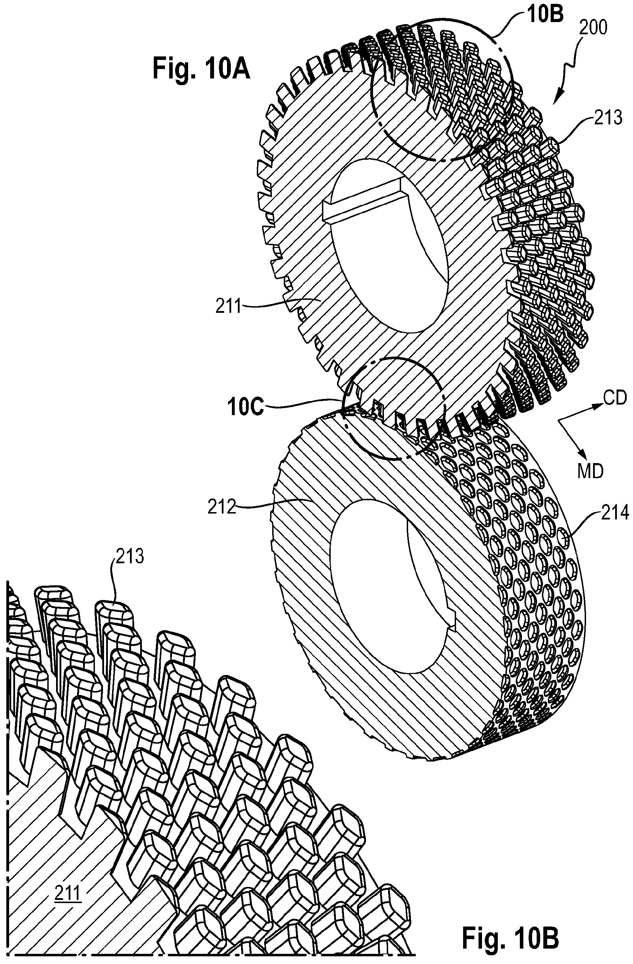

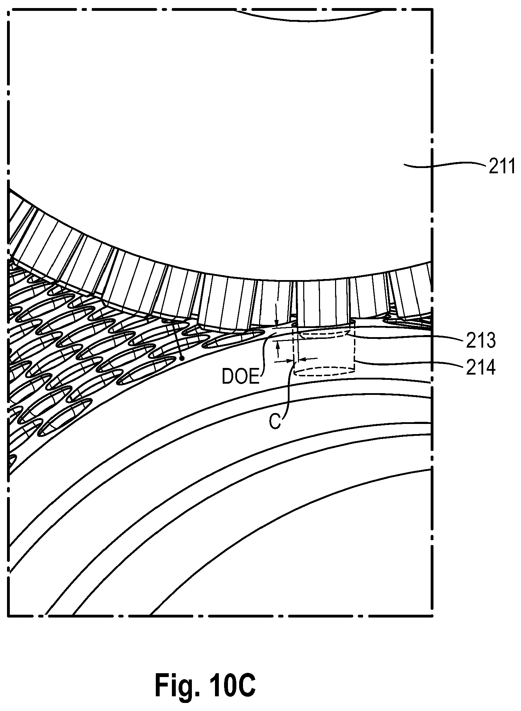

The topsheet 24 and the acquisition layer 52 may be engaged together between a first and second forming members (211, 212) and be simultaneously mechanically deformed and combined together to form the topsheet/acquisition layer laminate 245, as exemplified in Figs. 10A, 10B and 10C. The topsheet/acquisition layer laminate 245 comprises thus deformations forming three-dimensional protrusions 250.

The first and second forming member (211, 212) may be drum-shaped, generally cylindrical as shown in Figs. 10A, 10B and 10C, or plate-shaped.

The first forming member 211 of the apparatus 200 may have a surface comprising a plurality of discrete, spaced apart male forming elements 213 having a base that is joined to the first forming member 211, a top that is spaced away from the base, and sides that extend between the base and the top of the male forming elements 213. The male forming elements 213 may have a plan view periphery, and a height.

The top on the male forming elements 213 may have a rounded diamond shape, see for example Fig. 10B, with vertical sidewalls and a radiused or rounded edge at the transition between the top and the sidewalls of the male forming element 213.

The second forming member 212 may have a surface comprising a plurality of recesses 214 in the second forming member 212. The recesses 214 may be aligned and configured to receive the respective male forming elements 213 therein. Hence, each recess 214 of the second forming member 212 may be sufficiently large to be able to receive each respective male forming element 213 of the first forming member 211. The recesses 214 may have a similar shape as the male forming elements 213. The depth of the recesses 214 may be greater than the height of the male forming elements 213.

The first and second forming member 211, 212 may be further defined by a depth of engagement (DOE) which is a measure of the level of intermeshing of the first and second forming member 211, 212, as shown in Fig. 10C. The DOE may be measured from the tip of the male forming elements 213 to the outermost portion of the surface of the second forming member 212 which portions are not within a recess 214. The DOE may range from 1.5 mm to 5.0 mm or from 2.5 mm to 5.0 mm or from 3.0 mm to 4.0 mm.

The first and second forming member 211, 212 may be defined by a clearance between the first and second forming member 211, 212 as shown in Fig. 10C. The clearance is the distance between the side wall of the male forming element 213 and the side wall of the recess 214. The clearance may range from 0.1 mm to 2 mm or from 0.1 mm to 1.5 mm from 0.1 mm to 1 mm.

The topsheet 24 and the acquisition layer 52 may be therefore engaged together between the first and second forming members 211, 212 and be mechanically deformed and combined together to form the topsheet/acquisition layer laminate 245. The topsheet/acquisition layer laminate 245 comprises mechanical deformations forming three-dimensional protrusions 250.

The present method, however, differs from some embossing processes in which the top of the male elements compress the material to be embossed against the bottom of the female elements, thereby increasing the density of the region in which the material is compressed.

The topsheet/acquisition layer laminate 245 may be notionally divided into a first and second area. The first and/or second area of the topsheet/acquisition layer laminate 245 may comprise the majority of the three-dimensional protrusions 250 having different shapes.

Viewed from a cross-sectional view, i.e. in a Z-direction, the majority of the three-dimensional protrusions 250 may have any suitable shapes which include, but are not limited to: bulbous- shaped, conical-shaped and mushroom shaped.

Viewed from above, the majority of the three-dimensional protrusions 250 may have any suitable shapes which include, but are not limited to: circular, diamond-shaped, round diamond-shaped, U.S. football-shaped, oval-shaped, clover-shaped, triangular-shaped, tear-drop shaped and elliptical-shaped protrusions. The majority of the three-dimensional protrusions 250 may be non-circular.

The majority of the three-dimensional protrusions 250 may form, in conjunction, one or more graphics. Having graphics can support the caregiver's perception that the absorbent article is well able to absorb the liquid bodily exudates.

The majority of the three-dimensional protrusions 250 may have similar plan view dimensions in all directions, or the majority of the three-dimensional protrusions 250 may be longer in one dimension than another. The majority of the three-dimensional protrusions 250 may have different length and protrusion base width (measured according to the Protrusion Base Width Test Method set out below) dimensions. The majority of the three-dimensional protrusions 250 may, thus, have a ratio of length to protrusion base width. The ratio of length to protrusion base width can range from 10:1 to 1:10.

In some cases, it might be desired to have the majority of the three-dimensional protrusions 250 which are larger either in the machine or cross-machine direction. For this, the materials composing the topsheet 24 and acquisition layer 52 can be thus relatively more extensible either along the longitudinal axis versus the transversal axis of the absorbent article or vice versa.

The majority of the three-dimensional protrusion 252 may comprise a void area 253 which is the portion of the three-dimensional protrusion 251A which does not comprise any fibers or very little fibers. The majority of the three-dimensional protrusion 250 may be defined by a protrusion base width $WB_1$ of the base 256 forming an opening which is measured from two side walls of the inner portion 251A at the base 256. The majority of the three-dimensional protrusion 250 may be defined by a width $WD_2$ of the void area 253 which is the maximum interior width measured between two side walls of the inner three-dimensional protrusion 251A or which is the maximum diameter of the side wall of the inner three-dimensional protrusion 251A when the distal portion has a substantially circular shape. The maximum interior width $WD_2$ of the void area 253 at the distal portion may be greater than the protrusion base width $WB_1$ of the base 256 of the three-dimensional protrusion 250. The protrusion base width $WB_1$ of the base 256 of the majority of the three-dimensional protrusion 250 may range from 1.5 mm to 15 mm or from 1.5 mm to 10 mm or from 1.5 mm to 5 mm or from 1.5 mm to 3 mm. Measurements of the dimensions of the protrusion base width $WB_1$ of the base 256 and the width $WD_2$ of the distal portion 257 can be made on a photomicrograph. When the size of the protrusion base width $WB_1$ of the base 256 is specified herein, it will be appreciated that if the openings are not of uniform width in a particular direction, the protrusion base width, $WB_1$, is measured at the widest portion. Measurements of the protrusion base width $WB_1$ of the base 256 or the maximum interior width $WD_2$ of the void area 253 at the distal portion 257 can be made on a photomicrograph at 20X magnification.

As the plurality of fiber (254A, 254B) composing the majority of the three-dimensional protrusions 250 may be present in the one or more side walls 255 of the majority of the three-dimensional protrusions 250, the majority of the three-dimensional protrusions may not collapse on one side and close off the opening at the base 256 when compressive forces are applied on the topsheet/acquisition layer laminate 245. The opening at the base 256 may be maintained and may create a ring of increased opacity around the opening at the base 256 when the three-dimensional protrusion 250 has been compressed. Hence, the majority of the three-dimensional protrusion 250 can be preserved and remain visible to the consumer when viewing the absorbent article 20 from the topsheet 24. The majority of the three-dimensional protrusion 250 can be preserved after being subjected to any inherent compressive forces due to the process or the step of compressing the absorbent articles comprising the topsheet/acquisition layer laminate 245 prior to be filled in a packaging.

In other words, the majority of the three-dimensional protrusions 250 may have a degree of dimensional stability in the X-Y plane when a Z-direction force is applied to the majority of the three-dimensional protrusions 250. It is not necessary that the collapsed configuration of the majority of the three-dimensional protrusions 250 be symmetrical, only that the collapsed configuration prevent the majority of the three-dimensional protrusions 250 from flopping over or pushing back into the original plane of the topsheet/acquisition layer laminate 245. Without wishing to be bound to any particular theory, the wide base 256 and large cap 52 (greater than the protrusion base width of the base opening 44), combined with the lack of a pivot point, causes the three-dimensional protrusions 250 to collapse in a controlled manner (the large distal portion 257 prevents the three-dimensional protrusion 250 from flopping over and pushing back into the original plane of the topsheet/acquisition layer laminate 245). Thus, the majority of the three-dimensional protrusions 250 are free of a hinge structure that would otherwise permit them to fold to the side when compressed.

It may be desirable for at least one of the three-dimensional protrusions 250 in the topsheet/acquisition layer laminate 245 to collapse in a controlled manner described below under the 7 kPa load when tested in accordance with the Accelerated Compression Method set out below.

Alternatively, at least some, or in other cases, a majority of the three-dimensional protrusions 250 may collapse in the controlled manner described herein.

Alternatively, substantially all of the three-dimensional protrusions 250 may collapse in the controlled manner described herein. The ability of the three-dimensional protrusions 250 to collapse may also be measured under a load of 35 kPa. The 7 kPa and 35 kPa loads simulate manufacturing and compression packaging conditions. Wear conditions can range from 2 kPa or less up to 7 kPa.

Generally, the majority of the three-dimensional protrusions 250 may be configured to collapse in a controlled manner such that each base 256 forming an opening remains open, and the protrusion base width (measured according to the Protrusion Base Width Test Method below) of each base 256 forming an opening is greater than 0.5 mm after compression.

Preferably, the ratio of the circumference length of the three-dimensional protrusions 250 to the length of the opening at the base 256 is less than 4:1.

To measure the circumference length of the three-dimensional protrusions 250, the topsheet/acquisition layer laminate 245 comprising the three-dimensional protrusions is arranged so that the viewing direction is co-linear with the longitudinal axis (MD) of the three-dimensional protrusions. If necessary, a cross-section of the three-dimensional protrusions 250 can be obtained by cutting the three-dimensional protrusions perpendicular to the longitudinal axis using sharp scissors or a razor blade, taking care in preserving the overall geometry of the three-dimensional protrusions while cutting it.

As shown in Fig.11B, the circumference length of the three-dimensional protrusions 250 are measured and recorded by starting the measurement at a first origination point A, proceeding along the side walls 255 of the three-dimensional protrusions to the distal portion 257 of the three-dimensional protrusions 250 at a second point B (along the median path of the fibers) and terminating the measurement at the third origination point C. The length of the opening at the base 256 is measured and recorded parallel to the plane of the three-dimensional substrate 240 between the first origination point A and the third origination point C. The circumference length of the three-dimensional protrusions 250 are measured where the three-dimensional protrusions are not under any pressure or strain.

**Carded nonwoven fibrous web comprised by the acquisition layer**

The acquisition layer comprises a carded nonwoven fibrous web. The acquisition layer may consist of one or more of the carded nonwoven fibrous web(s). The fibers of the carded nonwoven fibrous web(s) are staple fibers.

The carded nonwoven fibrous web of the present invention comprises at least 50%, by weight of the carded nonwoven fibrous web, of staple fibers and at least 10%, by weight of the carded nonwoven fibrous web, of a latex binder. Staple fibers are short fibers, they may have a length of from 10 mm to 120 mm, or from 25 mm to 80 mm, or from 25 mm to 60 mm.

The carded nonwoven fibrous web may essentially consist of staple fibers and a latex binder, i.e. the carded nonwoven fibrous web may, in addition to the staple fibers and latex binder, consist of minor amounts of additives, such as odor control additives, perfumes, colored pigments or the like.

For the present invention, staple fibers laid down by a carding process form a layer of non-consolidated fibers. The layer then undergoes a through-air bonding process to form an autogenously bonded web.

Thereafter, a latex binder is applied on the autogenously bonded carded nonwoven fibrous web and the fibrous web with the binder applied thereon undergoes a curing step to cross-link the binder.

The basis weight of the carded nonwoven fibrous web may be from 20 to 100 g/m$^2$, or from 30 to 80 g/m$^2$, or from 35 to 70 g/m$^2$.

**Carding process**

Carding is a mechanical process using staple fibers. To obtain staple fibers, the fibers are first spun, cut to a few centimeters length, and put into bales (bundles of compressed fibers). The carding process starts with the opening of the bales of fibers which may be blended and are typically conveyed to the next stage by air transport. They are then combed into a web by a carding machine, such as a rotating drum or series of drums covered in fine wires or teeth. The precise configuration of cards will depend on the fabric weight and fiber orientation required. The web can be parallel-laid, where most of the fibers are laid in the direction of the web travel, or they can be random-laid. Typical parallel-laid carded webs result in good tensile strength, low elongation and low tear strength in the machine direction and the reverse in the cross direction.

In contrast to carded nonwoven webs, spunlaid and meltblown nonwoven webs are typically made in one continuous process. Fibers are spun and then directly dispersed into a web by deflectors or directed with air streams. The fibers of a spunlaid or meltblown nonwoven are considerably longer compared to staple fibers.

**Through air bonding**

As used herein, through-air bonding or "TAB" means a process of bonding staple fibers of the layer of non-consolidated fibers in which air is forced through the web, wherein the air is sufficiently hot to melt (or at least partly melt, or melt to a state where the fiber surface becomes sufficiently tacky) the polymer of a staple fiber or, if the staple fibers are multicomponent fibers, wherein the air is sufficiently hot to melt (or at least partly melt, or melt to a state where the fiber surface becomes sufficiently tacky) one of the polymers of which the fibers of the web are made. The air velocity is typically between 30 and 90 meter per minute and the dwell time may be as long as 6 seconds. The melting and resolidification of the polymer provides the bonding between different staple fibers.

A through air bonder is schematically shown in Fig. 26. In the through-air bonder 70, air having a temperature above the melting temperature of the polymer of the staple fiber or, if the staple fibers are multicomponent fibers, above the melting temperature of a first fiber component and below the melting temperature of a second fiber component, is directed from the hood 72, through the web, and into the perforated roller 74. Alternatively, the through-air bonder may be a flat arrangement wherein the air is directed vertically downward onto the web. The operating conditions of the two configurations are similar, the primary difference being the geometry of the web during bonding.

The hot air melts the staple fiber, or, for multicomponent fibers, the lower melting polymer component and thereby forms bonds between the staple fibers to consolidate and integrate the layer of staple fibers into a web.

As an example for a bicomponent fiber, when polypropylene and polyethylene are used as polymer components A and B respectively, the air flowing through the through-air bonder usually has a temperature ranging from about 110°C to about 162°C at a velocity from about 30 to about 90 meters per minute. It should be understood, however, that the parameters of the through-air bonder depend on factors such as the type of polymers used and thickness of the fibrous layer.

## Latex binder

The carded nonwoven fibrous web of the present invention comprises at least 10%, by weight of the carded nonwoven fibrous web, of a latex binder. The carded nonwoven fibrous web of the present invention may comprise at least 15%, or at least 20%, or at least 25%, or at least 30%, by weight of the carded nonwoven fibrous web, of a latex binder. The carded nonwoven fibrous web of the present invention comprises less than 50%, by weight of the carded nonwoven fibrous web, of a latex binder, and may comprise less than 45%, or less than 40%, or less than 35%, by weight of the carded nonwoven fibrous web, of a latex binder.

A suitable latex binder is prepared by a process including the steps of:

(1) polymerizing a monomer mixture comprising styrene, itaconic acid, surfactant and water soluble free radical initiator to form a seed;

(2) sequentially adding equal increments of a monomer mixture of styrene, butadiene and acrylic acid to the seed under emulsion polymerization conditions to form a styrene-butadiene-acrylic acid copolymer; and then

(3) neutralizing the styrene-butadiene-acrylic acid copolymer to a pH of about 4.5 to 7 to form the latex binder.

The binder is applied onto the autogenously bonded carded fibrous web. Subsequently, the latex binder is cured, using methods well known in the art, such as by application of heat or radiation. The term "cured" refers to the latex binder being cross-linked. The curing of the treated staple fibers is affected by a temperature above the glass transition temperature of the binder.

The latex binder may be prepared by well-known conventional emulsion polymerization techniques using one or more ethylenically unsaturated monomers and a polymeric surfactant as herein disclosed and additional conventional additives such as free-radical initiators, optional chain transfer agents, chelating agents and the like can be utilized as set forth in U.S. Pat. No. 5,166,259 to Schmeing and White.

In accordance with a preferred embodiment, the latex is prepared by polymerizing a monomer mixture comprising styrene, itaconic acid, surfactant and a water soluble free radical initiator to form a seed. A monomer mixture is then added incrementally to the seed under emulsion polymerization conditions. The monomer mixture includes styrene, butadiene, and acrylic acid. The acrylic acid can help in the cross-linking process of the binder upon curing. The monomer mixture is preferably added incrementally to the seed to form a styrene-butadiene-acrylic acid copolymer. In a preferred embodiment, the monomer mixture includes about 34-70 wt % styrene of the total composition. The monomer mixture also includes about 0.5-2.5 wt % itaconic acid, preferably 2 wt % itaconic acid of the total composition, about 20-55 wt % butadiene and acrylic acid in an amount of about 6-10 wt %, preferably 8 wt %.

A surfactant is added to the monomer mixture in an amount of about 0.05-2.0 wt %. The surfactant may be most any suitable emulsifier, soap, or the like well known in the art and suitable at the pH of the latex. Examples of suitable emulsifiers and surfactants include alkyl sulfates, alkyl sulfosuccinates, alkyl aryl sulfonates, alpha-olefin sulfonates, fatty or rosin acid salts, only or octyl phenol reaction products of ethylene oxide and the like. Other surfactants that may be used include those identified in Surface Active Agents, Schwartz and Berry, Vol. 1, Interscience Publishers, Inc., New York, 1958; Surface Activity, Moilet, Collie and Black, D. Van Nostrand Company, Inc., New York, 1961; Organic Chemistry, Feiser and Feiser, D.C. Heath and Company, Boston, 1944; and The Merck Index, Seventh Edition, Merck & Co., Inc., Rahway, N.J., 1960, all of which are hereby incorporated by reference.

The copolymer is then neutralized to a pH of about 4.5 to 7.0 to form the latex. The pH of the latex is neutralized by addition of a base. Examples of a suitable base include potassium hydroxide, sodium bicarbonate, ammonium hydroxide, sodium hydroxide and the like. The amount of base added to the latex is adjusted to obtain the desired pH range as is well known in the art.

Polymerization is typically carried out from about 65°C to 75°C. Polymerization is generally conducted for about 4 to 24 hours, however polymerization conditions may vary as desired to provide different conversion levels of monomer to copolymer. The monomer mixture is allowed to react until substantially constant solids at which time at least 99% of the monomers have been converted.

## The staple fibers

The carded nonwoven fibrous web comprised by the acquisition layer of the present invention comprises at least 50%, by weight of the carded nonwoven fibrous web, of staple fibers. The carded nonwoven fibrous web of the present invention may comprise at least 55%, or at least 60%, or at least 65%, or at least 70%, by weight of the carded nonwoven fibrous web, of staple fibers. The carded nonwoven fibrous web of the present invention comprises less than 90%, by weight of the carded nonwoven fibrous web, of staple fibers, and may comprise less than 85%, or less than 80%, or less than 75%, by weight of the carded nonwoven fibrous web, of staple fibers.

The fibers useful for the carded nonwoven fibrous web of the present invention are monocomponent fibers as well as multicomponent fibers. Multicomponent fibers are especially useful. Suitable multicomponent fibers are bicomponent fibers, such as core/sheath bicomponent fibers and side-by-side bicomponent fibers. The core/sheath bicomponent fibers may be concentric or eccentric fibers.

The monocomponent or multicomponent fibers may be made of polymeric materials, such as polyolefins (e.g. polypropylene, or polyethylene), polyester, polyethylene terephthalate (PET), CoPET, polybutylene terephthalate, polyamide, polylactic acid, viscose, and combinations thereof. The polymers may also comprise copolymers such as Co-PET. If the staple fibers comprise core/sheath bicomponent fibers, it is desirable that the sheath is made of a polymer which has a melting point below the melting point of the polymer which forms the sheath. If such bicomponent fibers are subjected to through-air bonding, the temperature of the through air bonding process is selected such that the polymer of the sheath is at least partially transferred to a molten state (or partly molten state, or molten to a state where the fiber surface becomes sufficiently tacky) such that the fibers bond together while the core of the bicomponent fiber remains substantially unaffected.

If side-by-side bicomponent fibers are used, the polymers forming the first and second component may also have different melting points. If such bicomponent fibers are subjected to through-air bonding, the temperature of the through air bonding process is selected such that the polymer of the component having the lower melting point is molten is at least partially transferred to a molten state (or partly molten state, or to a state where the fiber surface becomes sufficiently tacky) such that the fibers bond together while the polymer of the component having the higher melting point remains substantially unaffected.

The carded nonwoven fibrous web may comprise a mixture of different types of fibers, such as a mixture of monocomponent fibers and bicomponent fibers. The staple fibers of the carded nonwoven fibrous web may comprise at least 20%, or at least 35%, or at least 50%, or at least 60%, or at least 70%, or at least 80%, by total weight of the staple fibers, of multicomponent fibers, such as core/sheath or side-by-side bicomponent fibers. The staple fibers may also consist only of multicomponent fibers, such as bicomponent fibers. The staple fibers may be a mixture of different multicomponent fibers, e.g. a mixture of different bicomponent fibers.

The carded nonwoven fibrous web may also comprise monocomponent fibers. For example, the staple fibers may comprise at least 5%, or at least 10%, or at least 15%, or at least 20%, or at least 25%, by total weight of the staple fibers, of monocomponent fibers. The carded nonwoven fibrous web may not comprise more than 50%, or not more than 40%, or not more than 30%, by total weight of the staple fibers, of monocomponent fibers.

The staple fibers of the carded nonwoven fibrous web may consist of a mixture bicomponent fibers (such as core/sheath bicomponent fibers or side-by-side bicomponent fibers) and monocomponent fibers, such that the bicomponent fibers and the monocomponent fibers together form 100% of the total weight of the staple fibers.

The shape of the staple fibers of the carded nonwoven fibrous web may be round (i.e. fibers having a circular cross-section). Alternatively, the staple fibers may have non-round shape, such as multilobal fibers (e.g. trilobal fibers), flat fibers ("ribbon-like" cross-section), rhomboid fibers or triangular fibers. In multilobal fibers, a central section is encircled by a multiplicity of lobes. E.g. in a trilobal fiber, the central section is encircled by three lobes.

The staple fibers may comprise or consist of a mixture of solid, round bicomponent fibers (such as core/sheath or side-by-side bicomponent fibers) and solid, multilobal (such as trilobal) monocomponent fibers. Alternatively, the staple fibers may comprise or consist of a mixture of solid, round bicomponent fibers (such as core/sheath or side-by-side bicomponent fibers) and solid, round monocomponent fibers.

Overall, for the carded nonwoven fibrous web of the present invention, which has been subjected to through air bonding prior to application of the latex binder, the latex binder has been found to distribute more evenly over the fiber surfaces throughout the carded nonwoven fibrous web and shows a reduced tendency to "collect" in clusters at the crosspoints of the staple fibers compared to resin-bonded carded webs without autogenous bonds.

At least 50%, or at least 60%, or at least 65%, or at least 70%, or at least 75% of the total weight of the staple fibers may have a first diameter (= first type of fibers). The first type of fibers may constitute all of the fibers of the carded nonwoven fibrous web, or may constitute not more than 95%, or not more than 90%, or not more than 85% of the total weight of the staple fibers. The staple fibers of the carded nonwoven fibrous web may further comprise at least 5%, or at least 10%, or at least 15%, or at least 20%, or at least 25% of the total weight of the staple fibers, fibers having a second diameter fiber (= second type of fibers), which is smaller than the diameter of the first type of The second type of fibers may constitute not more than 35%, or not more than 30%, or not more than 25% of the total weight of the staple fibers. The first and second type of fibers together may constitute 90%, or 95%, or 100% of the total weight of the staple fibers of the carded nonwoven fibrous web.

The diameter of the first type of fibers may be at least 50% larger, or at least 60% larger, or at least 80% larger, or at least 100%, or at least 200% larger than the diameter of the first type of fibers. The diameter of the first type of fibers may be from 3 to 10 denier, or from 4 to 8 denier. The diameter of the second type of fibers may be from 0.8 denier to 2.5 denier, or from 1.0 denier to 2.0 denier.

Having a relatively high percentage of fibers with relatively high fiber diameter (e.g. from 3 to 10 denier) provides carded nonwoven fibrous webs with a relatively high degree of porosity. Such high degree of porosity is especially desirable for use of the material as acquisition material in an absorbent article.

Compared with the through air bonding applied for the carded nonwoven fibrous web of the present invention, the use of spunlace material, in which a carded fibrous layer is subjected to hydroentagling to consolidate the web and impart integrity, typically requires fibers with a relatively low fiber diameter. The low fiber diameter is needed as the fibers need to be able to bend sufficiently in order to entangle the fibers with each other. However, making carded nonwoven fibrous webs made of low denier fibers generally leads to webs with relatively low porosity, which makes them less attractive for use as acquisition material in absorbent articles.

Moreover, the through air bonding provides for autogenous bond sites which are relatively stable versus hydroengangled webs with no fiber to fiber bonds. During the application of latex binder and subsequent curing, the web is typically strained as it travels e.g. between calendar rolls. In the absence of stable fiber to fiber bonds (i.e. the autogenous bond sites), the fibers tend to rearrange and the web often loses a considerable portion of its initial loftiness, leading to a flatter web with reduced void volume. Hence, the combination of autogenous fiber to fiber bonds with the application of a latex binder has been found to be especially advantageous for webs to be used as acquisition layer in absorbent articles.

Having a certain amount of staple fibers with rather small fiber diameter helps to increase overall opacity of the web, especially if shaped fibers, such as multilobal (e.g. trilobal) fibers are applied, given that non-round fibers have a higher ratio of fiber surface area to fiber volume.

Increased opacity is desirable as it helps to disguise body exudates beneath the acquisition layer (towards the backsheet). Moreover, increased opacity also can increase the visibility of the three-dimensional protrusions of the topsheet/acquisition layer laminate. While fibers with a rather small fiber diameter can increase opacity of the web, it has been found that they do not negatively impact the resiliency (i.e. improved caliper recovery upon compression).

### The carded nonwoven fibrous web with autogenous bonds and latex binder

It has been found that compared to carded nonwoven fibrous webs which have solely undergone through-air bonding to consolidate the staple fibers into a coherent web, the additional, subsequent application of a latex binder facilitates nonwoven fibrous webs with improved caliper recovery after compression at different pressure levels.

For example (see Examples below), upon compression at 12 kPa, the carded nonwoven fibrous web (not having been combined with a topsheet into a laminate having three-dimensional protrusions) show a caliper improve in compression recovery of at least 5 % or at least 7%, compared to a carded, through-air bonded nonwoven fibrous web without additional latex bonding. A pressure of up to 12 kPa may occur for highly compressed absorbent articles in packages.

Typical use conditions of absorbent articles may subject the article to pressures of about 1 kPa or up to 4 kPa upon pressure exerted by the wearer. Upon such pressures, it has been found that the carded nonwoven fibrous webs of the present invention show a caliper improvement in compression recovery (upon compression at 4 kPa) of at least 5 % or at least 7%; or, upon compression at 1 kPa, of at least 4% compared to a carded, through-air bonded nonwoven fibrous web without additional latex bonding.

Moreover, air permeability of carded nonwoven webs with autogenous bonds and latex binder has shown to increase (for webs which have not undergone compression) by at least 4%, or by at least 10%, or by at least about 20%, or by at least about 30% compared to a carded, through-air bonded nonwoven fibrous web without additional latex bonding (see Examples below).

Also, permeability, measured by In Plane Radial Permeability (IPRP) has been found to increase (for webs which have not undergone compression) by at least 10%, or at least 20%, or at least 25% compared to a carded, through-air bonded nonwoven fibrous web without additional latex bonding (see Examples below).

Moreover, it has been found that IPRP is not only improved for the carded nonwoven fibrous web which has not undergone compression but is also improved for carded nonwoven fibrous webs which have been previously compressed, compared to a carded, through-air bonded nonwoven fibrous web without additional latex bonding (see Examples below).

Permeability, measured by In Plane Radial Permeability (IPRP) has been found to increase (for webs 24 h after compression at 4 kPa) by at least 10%, or at least 20%, or at least 25% compared to a carded, through-air bonded nonwoven fibrous web without additional latex bonding (see Examples below).

Hence, the carded nonwoven fibrous webs used to be subsequently joined with a topsheet in order to form a laminate with three-dimensional protrusions, have been found to have improved properties versus known carded fibrous webs without latex binder, especially with regard to properties important for use as acquisition layers of such fibrous webs in absorbent articles.

Compared to known carded nonwoven fibrous webs which have only been consolidated by use of a latex binder without having also undergone through-air bonding to form autogenous bonds between staple fibers, the fibrous web, used to be subsequently joined with a topsheet in order to form a laminate with three-dimensional protrusions, may have good void volume: Upon curing the nonwoven webs to cross-link the binder, the nonwovens are typically subjected to tension, e.g. as they travel between a pair of rolls (to stabilize their position along the manufacturing line). If the staple fibers have not been bonded to each other to form a coherent web prior to this process, the staple fibers tend to re-arrange their position within the fibrous layer, which may reduces loft and thus, void volume of the subsequently obtained web.

Further to providing improved void volume, such carded nonwoven fibrous webs having autogenous fiber bonds in addition to being consolidated with a latex binder, may have improved softness due to their improved loftiness, when compared to a carded nonwoven fibrous web with no autogenous bonds.

Furthermore, upon being subjected to tension in MD in the manufacturing process, the re-arrangement of the non-consolidated staple fibers can result in a relatively high degree of fiber orientation in MD. Typically, fiber orientation of the staple fibers in carded nonwoven fibrous webs with only latex bonding is in the range of 5 / 1 (MD / CD) up to 10 / 1 (MD / CD). This relatively high degree of fiber orientation results in relatively high tensile strength of the web in MD compared to the tensile strength in CD. When such webs are joined to a topsheet by mechanical deformation in order to form a laminate with three-dimensional protrusions, they may show a relatively high tendency to necking when subjected to MD tension (i.e. they become narrower in CD). This necking can pose problems in the process of joining the nonwoven fibrous web to the topsheet to form the laminate.

By subjecting the non-consolidated staple fibers to through-air bonding prior to application of the latex binder, the nonwoven non-consolidated staple fibers can be transformed into a self-sustaining web relatively quickly and before the web undergoes strain in the curing process step to cross-link the binder. Due to the autogenous bonds between the staple fibers, subsequent fiber re-arrangement can be reduced and hence, the carded nonwoven fibrous webs of the present invention can be facilitated with less fiber orientation in MD (i.e. with more randomly arranged fibers).

**Method of making the carded nonwoven fibrous web (which is used as acquisition layer and subsequently incorporated into the topsheet/acquisition layer laminate)**

The method of making the carded nonwoven fibrous web, which is subsequently used to be subsequently joined with a topsheet in order to form a laminate with three-dimensional protrusions, may comprise the steps of:

In a first step, a layer of staple fibers is formed. Staple fibers are formed by a carding process and laid down on a conveyor belt or drum to form a layer of non-consolidated staple fibers. The layer is preferably laid down in form of a continuous layer. The layer of non-consolidated fibers maybe a homogeneous layer having substantially homogeneous basis weight. "Substantially homogeneous basis weight" is to be understood, in the sense that the basis weight may vary slightly due to process conditions, especially for relatively low basis weight fiber lay down; however, the basis weight is not varied intentionally throughout the layer of non-consolidated staple fibers. Alternatively, the basis weight may vary across CD and/or MD, i.e. the basis weight differs intentionally in CD and/or MD.

In a subsequent method step, the layer of non-consolidated staple fibers is subjected to air-through bonding. Through air bonders are described in more detail above. Due to the air-through bonding, the staple fibers are autogenously bonded to each other. As a result of the air-through bonding, a consolidated nonwoven fibrous web with relatively high loft is formed.

Thereafter, a liquid latex binder is applied onto the consolidated nonwoven fibrous web. The latex binder is preferably applied homogeneously onto one of the two surfaces of the fibrous web. The ratio of staple fibers to latex binder is from 90/10 to 60/40 (by weight of the staple fibers and binder). The ratio of staple fibers to latex binder maybe from 90/10, or from 85/15, or from 80/20 or from 75/25, up to 60/40, or up to 65/45, or up to 70/30. The binder may be applied by known methods, such as by directing the consolidated nonwoven fibrous web through a bath or basin, which contains the liquid latex binder (also referred to as "kiss roll application", by spraying the liquid latex binder onto the consolidated nonwoven fibrous web, or by foam application, whereby the liquid latex binder is in a foamed state, with which the fibrous web is impregnated.

Then, the web with the binder applied thereon is cured at an elevated temperature sufficient to cross-link the binder (however, not as high as to melt the staple fibers) to obtain the carded nonwoven fibrous web of the present invention.

Alternatively, though less desirable, it is also possible to apply the liquid latex binder onto a layer of non-consolidated staple fibers which have not previously undergone air-through bonding. The layer of non-consolidated fibers with the latex binder applied thereon is then subjected to air-through bonding to autogenously bond the fibers to each other and subsequently to a curing step at an elevated temperature sufficient to cross-link the binder (however, not as high as to melt the staple fibers) to obtain the carded nonwoven fibrous web of the present invention.

In still another, less desirable alternative, the liquid latex binder is applied onto a layer of non-consolidated staple fibers which have not previously undergone air-through bonding. The layer of non-consolidated fibers with the latex binder applied thereon is then subjected to a curing step at an elevated temperature sufficient to cross-link the binder (however, not as high as to melt the staple fibers) to obtain the carded nonwoven fibrous web of the present invention, and, subsequently, to air-through bonding to autogenously bond the fibers to each other.

Generally, the process has to be done such as to consider the melting point of the fibers (or the sheath of the fibers, if bicomponent fibers are used) and the curing temperature of the latex binder.

It may also be possible, though less desirable, to cure the latex binder and autogenously bond the staple fibers to each other in one process step if the melting point of the fibers (or the sheath of fibers, if bicomponent fibers are used) and the curing temperature of the latex binder are in the same range. Notably, this is different from the known curing of latex binders applied on nonwoven webs, which do not use temperatures at which the fibers would melt and bond to each other.

The web maybe wound up in roll form for storage or transport. Alternatively, the web may be directly conveyed to a subsequent method step, such as the step where the web is combined with a topsheet to form the topsheet/acquisition layer laminate of the present invention.

Topsheet materials

Several examples of nonwoven materials suitable for use as a topsheet 24 for the topsheet/acquisition layer laminate 245 may include, but are not limited to: spunbonded nonwovens; carded nonwovens; and nonwovens with relatively specific properties to be able to be readily deformed.

One suitable nonwoven material as a topsheet 24 for the topsheet/acquisition layer laminate 245 may be an extensible polypropylene/polyethylene spunbonded nonwoven. One suitable nonwoven material as a topsheet 24 for the topsheet/acquisition layer laminate 245 may be a spunbonded nonwoven comprising polypropylene and polyethylene. The fibers may comprise a blend of polypropylene and polyethylene. Alternatively, the fibers may comprise bi-component fibers, such as a sheath-core fiber with polyethylene on the sheath and polypropylene in the core of the fiber.

The topsheet 24 of the topsheet/acquisition layer laminate 245 may have a basis weight from 8 to 40 gsm or from 8 to 30 gsm or from 8 to 20 gsm.

The topsheet 24 and acquisition layer 52 may be joined together prior or during the mechanical deformation. If desired an adhesive, chemical bonding, resin or powder bonding, or thermal bonding between the topsheet 24 and acquisition layer 52 may be selectively utilized to bond certain regions or all of the topsheet 24 and acquisition layer 52 together.

Prior to any mechanical deformation, the topsheet 24 may be attached to the acquisition layer 52. For instance, the topsheet 24 may be attached to the acquisition layer 52 where the topsheet 24 and acquisition layer 52 overlaps. The attachment of the topsheet 24 to the acquisition layer 52 may include a uniform continuous layer of adhesive, a discontinuous patterned application of adhesive or an array of separate lines, spirals, or spots of adhesive. The basis weight of the adhesive in the topsheet/acquisition layer laminate 245 may be from 0.5 to 20 $g/m^2$ or from 1 to 10 gsm or from 2 to 5 $g/m^2$.

Delamination of binder

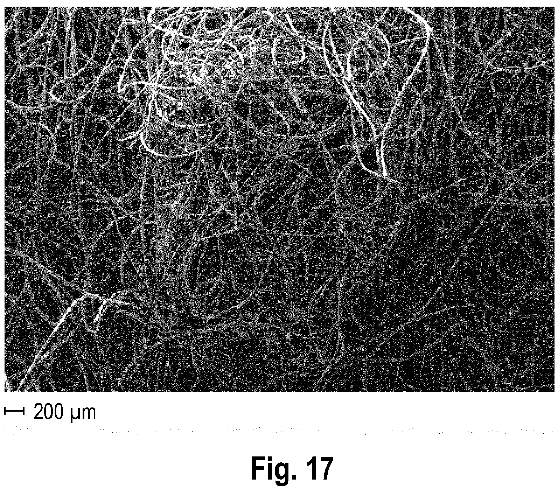

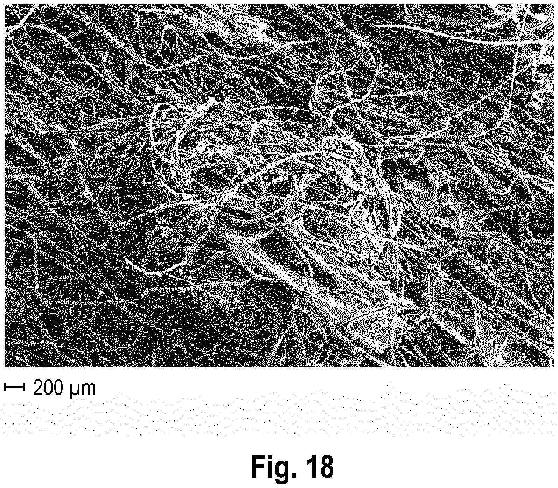

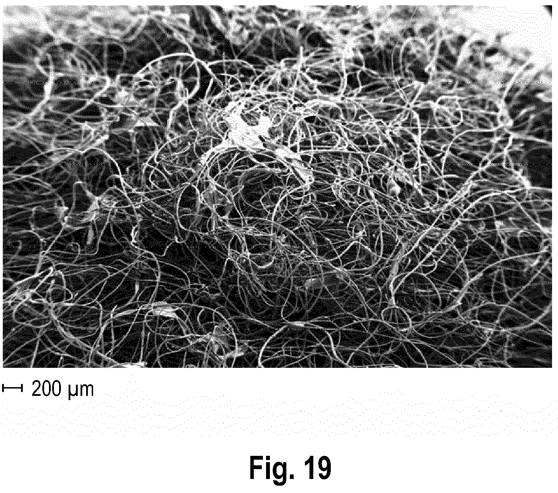

It has been surprisingly found that when using the carded nonwoven fibrous web which has been autogenously bonded and subsequently treated with latex binder, as described in detail above, the binder exhibits a larger degree of delamination from the staple fibers in the three-dimensional protrusions of the topsheet/acquisition layer laminate than in the plane areas of the topsheet/acquisition layer laminate (shown in Fig. 17). Figs. 18 and 19 show SEM pictures of topsheet/acquisition layer laminates wherein the acquisition layer has only been bonded by use of a latex binder (i.e. no autogenous bonds). Fig. 18 shows the topsheet/acquisition layer laminate of Comparative Example C (described below) and Fig. 19 shows the topsheet/acquisition layer laminate of Comparative Example D (described below). As can be seen, there is substantially no delamination of the binder from the fibers in the area of the protrusion. Hence, the pictures illustrate the difference with respect to binder delamination between carded nonwoven fibrous webs with and without autogenous bonds. The SEM pictures in Fig. 17, 18 and 19 show the surface of the laminate which is formed by the acquisition layer.

It is believed that this effect contributes to the reduced loss in Protrusion Height after compression (which relates to reduced loss of void volume) of the topsheet/acquisition layer laminate compared to laminates using acquisition layers with only autogenous bonds or only latex binder (see Example A and Comparative Examples A-D below). The strain which the fibers undergo during the mechanical deformation, is believed to be "absorbed" partly by the binder which forms a kind of film layer on the fiber surface. Hence, upon mechanical deformation, this "binder film" delaminates from the fiber rather than leading to tearing of the fibers. As it has been found that a carded nonwoven fibrous web which has only been consolidated by use of latex, the binder does not apply as evenly over the fiber surface compared to use of latex binder on autogenously bonded carded nonwoven fibrous webs, this effect of binder delamination cannot be observed.

The topsheet/acquisition layer laminate of the present invention may have a loss of Protrusion Height of less than 25%, or less than 23%, or less than 22%, or less than 20% after compression with an applied pressure of 4 kPa according to the test method set out herein.

The topsheet/acquisition layer laminate hay have a caliper recovery of at least 70%, or at least 75%, after compression with an applied pressure of 4 kPa according to the test method set out herein.

## Test Methods

Unless indicated otherwise, all tests described herein are made with samples conditioned at least 24 hours at 23 °C +/- 2 °C and 50% +/- 10% Relative Humidity (RH).

## Caliper method

The caliper of the material sample is measured using a dial gauge or digital equivalent with a resolution of $\pm$ 10 $\mu$m and a circular "foot" having a flat bottom circular surface with a diameter of 56mm. The gauge is mounted over a base having a horizontal flat rigid upper surface, such that the entire bottom surface of the foot contacts the upper surface of the base.

The downward force exerted by the foot on the base or on a material sample inserted between the foot and the base is depending on the weight of the foot, i.e. depending on the exact equipment used.

The weight exerted by the foot of the gauge can be measured by mounting the gauge over a suitable top-loading balance such that the balance pan is in the same relative position to the gauge as the base. It is independent of the caliper of the material sample. The force is adjusted by adding weight to the foot such that the total weight is 518 g, i.e. the pressure exerted by the foot of 56 mm diameter is 2065 $\pm$ 10 Pa.

The gauge is calibrated according to the manufacturer's instructions.

The material sample is cut as a circle of 6 cm diameter. Such material sample is placed on the base such that the foot is completely in contact with the material sample. If the material to be tested is the topsheet/acquisition layer laminate, the sample is cut from the laminate and the laminate sample is tested.

The caliper of the material sample is determined by reading the gauge with the foot resting on the base (GO). The foot of the gauge is then raised and the material is laid flat on the base. The foot is lowered gently onto the material sample and the gauge reading is taken 5 seconds after the foot comes into contact with the sample (GT). The caliper of the material sample at that location is the difference between the two readings (GT-G0). The caliper is the average of three replicates and is reported in millimeters rounded to the nearest 0.01mm.

## Compression at 4kPa and calculation of caliper recovery

Samples having size of 8x8 cm are cut from the carded nonwoven fibrous web. 10 samples are laid down on top of each other in a face to face relationship. A circular weight of 1150 k and having a diameter of 6 cm is used exerting a pressure of 4 kPa is then placed on top of the pile of 10 samples and is left on the pile for 15 hours at 40°C and 75% relative humidity (RH).

The caliper of each of the 10 sample is measured separately prior to putting the weight on the samples, immediately after the weight is removed and 24 hours after the weight is removed. During the 24 hours after the weight is removed, the samples are stored at 22°C and 50% RH. The average of the 10 caliper measurements, respectively taken prior to applying the weight, immediately after removing the weight and 24 hours after removing the weight, is taken to report caliper.

Caliper recovery [%] is calculated as:

$$\frac{\underline{\text{Caliper 24 h after weight is removed x 100}}}{\text{Caliper before compression}}$$

**In Plane Radial Permeability (IPRP)**

In plane radial permeability or IPRP or shortened to permeability in the present invention is a measure of the permeability of the nonwoven fabric and relates to the pressure required to transport liquids through the material. The following test is suitable for measurement of the In-Plane Radial Permeability (IPRP) of a porous material. The quantity of a saline solution (0.9% NaCl) flowing radially through an annular sample of the material under constant pressure is measured as a function of time. (Reference: J.D. Lindsay, "The anisotropic Permeability of Paper" TAPPI Journal, (May 1990, pp. 223) Darcy's law and steady-state flow methods are used for determining in-plane saline flow conductivity).

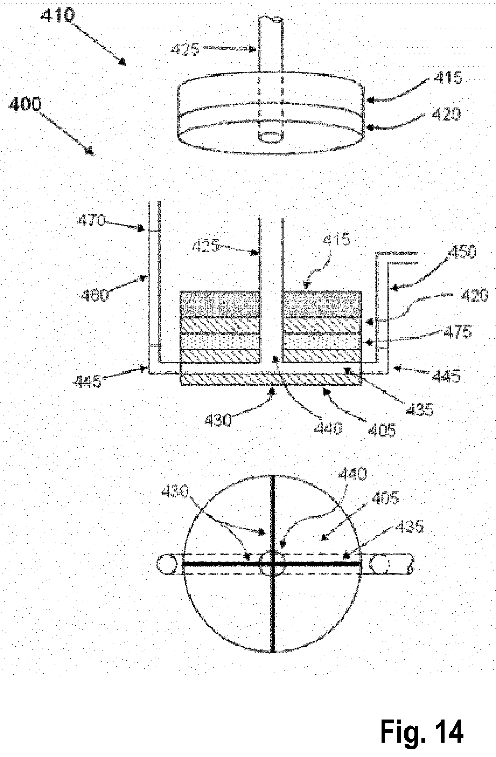

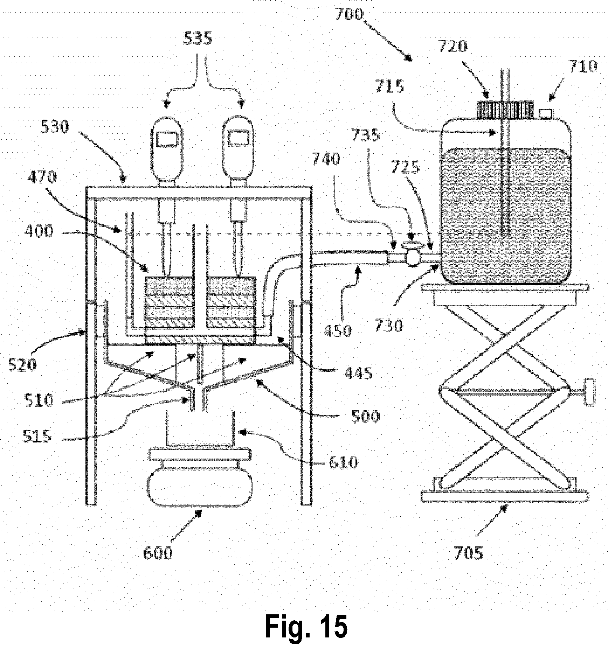

The IPRP sample holder 400 is shown in FIG. 14 and comprises a cylindrical bottom plate 405, top plate 420, and cylindrical stainless steel weight 415 shown in detail in FIG. 15.

Top plate 420 is 10 mm thick with an outer diameter of 70.0 mm and connected to a tube 425 of 190 mm length fixed at the center thereof. The tube 425 has in outer diameter of 15.8 mm and an inner diameter of 12.0 mm. The tube is adhesively fixed into a circular 12 mm hole in the center of the top plate 420 such that the lower edge of the tube is flush with the lower surface of the top plate, as depicted in FIG. 15. The bottom plate 405 and top plate 420 are fabricated from Lexan® or equivalent. The stainless steel weight 415 has an outer diameter of 70 mm and an inner diameter of 15.9 mm so that the weight is a close sliding fit on tube 425. The thickness of the stainless steel weight 415 is approximately 25 mm and is adjusted so that the total weight of the top plate 420, the tube 425 and the stainless steel weight 415 is 788 g to provide 2.1 kPa of confining pressure during the measurement.

As shown in FIG. 15, bottom plate 405 is approximately 50 mm thick and has two registration grooves 430 cut into the lower surface of the plate such that each groove spans the diameter of the bottom plate and the grooves are perpendicular to each other. Each groove is 1.5 mm wide and 2 mm deep. Bottom plate 405 has a horizontal hole 435 which spans the diameter of the plate. The horizontal hole 435 has a diameter of 11 mm and its central axis is 12 mm below the upper surface of bottom plate 405. Bottom plate 405 also has a central vertical hole 440 which has a diameter of 10 mm and is 8 mm deep. The central hole 440 connects to the horizontal hole 435 to form a T-shaped cavity in the bottom plate 405. The outer portions of the horizontal hole 435 are threaded to accommodate pipe elbows 445 which are attached to the bottom plate 405 in a watertight fashion. One elbow is connected to a vertical transparent tube 460 with a height of 190 mm and an internal diameter of 10 mm. The tube 460 is scribed with a suitable mark 470 at a height of 50 mm above the upper surface of the bottom plate 420. This is the reference for the fluid level to be maintained during the measurement. The other elbow 445 is connected to the fluid delivery reservoir 700 (described below) via a flexible tube.

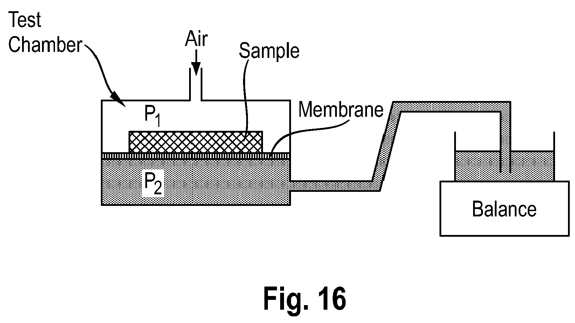

A suitable fluid delivery reservoir 700 is shown in FIG. 16. Reservoir 700 is situated on a suitable laboratory jack 705 and has an air-tight stoppered opening 710 to facilitate filling of the reservoir with fluid. An open-ended glass tube 715 having an inner diameter of 10 mm extends through a port 720 in the top of the reservoir such that there is an airtight seal between the outside of the tube and the reservoir. Reservoir 700 is provided with an L-shaped delivery tube 725 having an inlet 730 that is below the surface of the fluid in the reservoir, a stopcock 735, and an outlet 740. The outlet 740 is connected to elbow 445 via flexible plastic tubing 450 (e.g. Tygon®). The internal diameter of the delivery tube 725, stopcock 735, and flexible plastic tubing 450 enable fluid delivery to the IPRP sample holder 400 at a high enough flow rate to maintain the level of fluid in tube 460 at the scribed mark 470 at all times during the measurement. The reservoir 700 has a capacity of approximately 6 liters, although larger reservoirs may be required depending on the sample thickness and permeability. Other fluid delivery systems may be employed provided that they are able to deliver the fluid to the sample holder 400 and maintain the level of fluid in tube 460 at the scribed mark 470 for the duration of the measurement.

The IPRP catchment funnel 500 is shown in FIG. 14 and comprises an outer housing 505 with an internal diameter at the upper edge of the funnel of approximately 125 mm. Funnel 500 is constructed such that liquid falling into the funnel drains rapidly and freely from spout 515. A horizontal flange 520 around the funnel 500 facilitates mounting the funnel in a horizontal position. Two integral vertical internal ribs 510 span the internal diameter of the funnel and are perpendicular to each other. Each rib 510 is 1.5 mm wide and the top surfaces of the ribs lie in a horizontal plane. The funnel housing 500 and ribs 510 are fabricated from a suitably rigid material such as Lexan® or equivalent in order to support sample holder 400. To facilitate loading of the sample it is advantageous for the height of the ribs to be sufficient to allow the upper surface of the bottom plate 405 to lie above the funnel flange 520 when the bottom plate 405 is located on ribs 510. A bridge 530 is attached to flange 520 in order to mount a dial gauge 535 to measure the relative height of the stainless steel weight 415. The dial gauge 535 has a resolution of ± 0.01 mm over a range of 25 mm. A suitable digital dial gauge is a Mitutoyo model 575-123 (available from McMaster Carr Co., catalog no. 19975-A73), or equivalent. Bridge 530 has two circular holes 17 mm in diameter to accommodate tubes 425 and 460 without the tubes touching the bridge.

Funnel 500 is mounted over an electronic balance 600, as shown in Fig. 14. The balance has a resolution of ± 0.01 g and a capacity of at least 2000g. The balance 600 is also interfaced with a computer to allow the balance reading to be recorded periodically and stored electronically on the computer. A suitable balance is Mettler-Toledo model PG5002-S or equivalent. A collection container 610 is situated on the balance pan so that liquid draining from the funnel spout 515 falls directly into the container 610.

The funnel 500 is mounted so that the upper surfaces of ribs 510 lie in a horizontal plane. Balance 600 and container 610 are positioned under the funnel 500 so that liquid draining from the funnel spout 515 falls directly into the container 610. The IPRP sample holder 400 is situated centrally in the funnel 700 with the ribs 510 located in grooves 430. The upper surface of the bottom plate 405 must be perfectly flat and level. The top plate 420 is aligned with and rests on the bottom plate 405. The stainless steel weight 415 surrounds the tube 425 and rests on the top plate 420. Tube 425 extends vertically through the central hole in the bridge 530. The dial gauge 535 is mounted firmly to the bridge 530 with the probe resting on a point on the upper surface of the stainless steel weight 415. The dial gauge is set to zero in this state. The reservoir 700 is filled with 0.9% saline solution and re-sealed. The outlet 740 is connected to elbow 445 via flexible plastic tubing 450.

An annular sample 475 of the material to be tested is cut by suitable means. The sample has an outer diameter of 70 mm and an inner hole diameter of 12 mm. One suitable means of cutting the sample is to use a die cutter with sharp concentric blades.

The top plate 420 is lifted enough to insert the sample 475 between the top plate and the bottom plate 405 with the sample centered on the bottom plate and the plates aligned. The stopcock 735 is opened and the level of fluid in tube 460 is set to the scribed mark 470 by adjusting the height of the reservoir 700 using the jack 705 and by adjusting the position of the tube 715 in the reservoir. When the fluid level in the tube 460 is stable at the scribed mark 470 and the reading on the dial gauge 535 is constant, the reading on the dial gauge is noted (initial sample thickness) and the recording of data from the balance by the computer is initiated. Balance readings and time elapsed are recorded every 10 seconds for five minutes. After three minutes the reading on the dial gauge is noted (final sample thickness) and the stopcock is closed. The average sample thickness Lp is the average of the initial sample thickness and the final sample thickness expressed in cm.

The flow rate in grams per second is calculated by linear least squares regression fit to the data between 60 seconds and 300 seconds. The permeability of the material is calculated using the following equation:

*Calculation*

Data acquisition starts 60 seconds after beginning the test. The software collects the following data at interval of 20 seconds:

Mass of saline in the container on the balance

Thickness of the measured sample

Conductivity or In Plane Radial Permeability (IPRP) $K_r$ is calculated according to Equation #1:

$$\text{Equation \#1:}$$

$$K_r = \frac{k_r}{\mu}$$

Where: $K_r$     is the Conductivity or In Plane Radial Permeability (IPRP)

$k_r$        is the permeability according to Equation #2

$\mu$         is the liquid dynamic viscosity of saline 0.9% (value used here: 0.001 Pa*s)

Equation #2:

$$k_r = \frac{\sum_{i=1}^{max} k_{r,i}}{max}$$

Where: $k_{r,i}$    is the permeability at time i according to Equation #3

max    is the number of acquired data points

Equation #3:

$$k_{r,i} = \frac{(Q_i / \rho) * \mu * \ln(R_o / R_{in})}{2\pi * Lp_i * \Delta p_i}$$

Where:

$Q_i$    is the mass flow rate at time i according to equation #4

$\rho$    is the density of 0.9% saline (value used here 1.01 g/cm3)

$\mu$    is the liquid dynamic viscosity at 20 °C.

$R_o$    is the outer sample radius.

$R_{in}$    is the inner sample radius.

$Lp_i$    is the averaged sample thickness for time i according to Equation #5

$\Delta p_i$    is the pressure drop calculated according to Equation #6:

Equation #4:

$$Q_{(i)} = \frac{(g_{(i)} - g_{(i-1)})}{(t_{(i)} - t_{(i-1)})}$$

Where:

$t_{(i)}$    is the time i

$g_{(i)}$    is the fluid mass measured by the balance at time i

$i$    is the index

Equation #5:

$$Lp_i = \frac{(lp_{(i)} + lp_{(i-1)})}{2}$$

Where:

$lp_i$     is the sample thickness at time i (TMM software averages the readings of the two caliper gauges).

$$ \text{Equation } \#6 $$

$$ \Delta p_i = \left( \Delta P - \frac{Lp_i}{2} \right) * g * \rho $$

Where:

g     is the acceleration gravity (value used here: $9.81 m/s^2$)

$\Delta P$     is the hydrostatic head as displayed in the glass tube. Note that the hydrostatic head is of dimension length.

## Air Permeability

Air permeability is measured in accordance with Edana test method WSP 70.1 (08).

TEXTEST FX 3300 Air Permeability Tester apparatus or equivalent is used.

Test Head Model FX 3300-20 or equivalent is used. An area of $20 \ cm^2$ is tested.

The pressure drop is set to 125 Pa, orifice area is $38.3 \ cm^2$.

The instrument is calibrated according to manufacturer's instruction, and test is performed as specified in manufacturer's instructions.

Testing is performed in a conditioned room maintained at 23°C $\pm$ 2°C and 50%RH $\pm$ 2%.

## Protrusion Base Width and Protrusion Height Test Methods

1) General information

The Measured Protrusion Base Width and Measured Protrusion Height of the three-dimensional protrusions of the topsheet/acquisition layer laminate of an absorbent article are measured using a GFM Primos Optical Profiler instrument commercially available from GFMesstechnik GmbH, Warthestraße 21, D14513 Teltow/Berlin, Germany. Alternative suitable non-touching surface topology profilers having similar principles of measurement and analysis, can also be used, here GFM Primos is exemplified.

The GFM Primos Optical Profiler instrument includes a compact optical measuring sensor based on a digital micro mirror projection, consisting of the following main components:

a) DMD projector with 800 x 600 direct digital controlled micro-mirrors

b) CCD camera with high resolution (640 x 480 pixels)

c) Projection optics adapted to a measuring area of at least 30 x 40 mm

d) Recording optics adapted to a measuring area of at least 30 x 40 mm

e) A table tripod based on a small hard stone plate

f) A cold light source (an appropriate unit is the KL 1500 LCD, Schott North America, Inc., Southbridge, MA)

g) A measuring, control, and evaluation computer running ODSCAD 6.3 software

Turn on the cold-light source. The settings on the cold-light source are set to provide a color temperature of at least 2800K.

Turn on the computer, monitor, and open the image acquisition/analysis software. In the Primos Optical Profiler instrument, select "Start Measurement" icon from the ODSCAD 6.3 task bar and then click the "Live Image button".

The instrument is calibrated according to manufacturer's specifications using calibration plates for lateral (X-Y) and vertical (Z). Such Calibration is performed using a rigid solid plate of any non-shiny material having a length of 11 cm, a width of 8 cm and a height of 1 cm. This plate has a groove or machined channel having a rectangular cross-section, a length of 11 cm, a width of 6.000 mm and an exact depth of 2.940 mm. This groove is parallel to the plate length direction. After calibration, the instrument must be able to measure the width and depth dimensions of the groove to within ± 0.004 mm.

All testing is performed in a conditioned room maintained at 23 ± 2°C and 50+/-10% relative humidity. The surface to be measured may be lightly sprayed with a very fine white powder spray. Preferably, the spray is NORD-TEST Developer U 89, available from Helling GmbH, Heidgraben, Germany.

2) <u>Protrusion Base Width Test Method</u>

The topsheet/acquisition layer laminate is extracted from the absorbent article by attaching the absorbent article to a flat surface in a taut planar (i.e. stretched planar) configuration with the topsheet of the topsheet/acquisition layer laminate facing up. Any leg or cuff elastics are severed in order to allow the absorbent article to lie flat. Using scissors, two longitudinal cuts are made through all layers above the absorbent core (i.e. the core wrap) along the edges of the topsheet/acquisition layer laminate. Two transversal cuts are made through the same layers following the front and back waist edges of the absorbent article.

The topsheet/acquisition layer laminate and any other layers above the absorbent core are then removed without perturbing the topsheet/acquisition layer laminate. Freeze spray (e.g. CRC Freeze Spray manufactured by CRC Industries, Inc. 885 Louis Drive, Warminster, PA 18974, USA), or equivalent aid may be used to facilitate removal of the uppermost layers from the absorbent article. The topsheet/acquisition layer laminate is then separated from any other layers, including any carrier layer (e.g. a nonwoven carrier layer, a tissue layer), using freeze spray if necessary. If a distribution layer, e.g. a pulp containing layer is attached to the topsheet/acquisition layer laminate, any residual cellulose fibers are carefully removed with tweezers without modifying the acquisition layer.

The topsheet/acquisition layer laminate with three-dimensional protrusions (conditioned at a temperature of 23°C ± 2°C and a relative humidity of 50% ± 10% for at least 24 hours) namely "the specimen" is laid down on a hard flat horizontal surface with the body-facing side upward, i.e. the topsheet of the topsheet/acquisition layer laminate being upward. Ensure that the specimen is lying in planar configuration, without being stretched, with the specimen uncovered.

A nominal external pressure of 1.86 kPa (0.27 psi) is then applied to the specimen. Such nominal external pressure is applied without interfering with the topology profile measurement. Such an external pressure is applied using a transparent, non-shining flat Plexiglas® plate 200 mm by 70 mm and appropriate thickness (approximately 5 mm) to achieve a weight of 83g. The plate is gently placed on top of the specimen, such that the center point of the Plexiglas® plate is at least 40 mm away from any folds, with the entire plate resting on the specimen. A fold corresponds to a part of the absorbent article (e.g. the topsheet/acquisition layer laminate) where the absorbent article has been folded for packaging purposes.

Two 50 mm x 70 mm metal weights each having a mass of 1200 g (approximate thickness of 43 mm) are gently placed on the Plexiglas® plate such that a 70 mm edge of each metal weight is aligned with the 70 mm edges of the Plexiglas® plate. A metal frame having external dimensions of 70 mm x 80 mm and interior dimensions of 42 mm x 61 mm, and a total weight of 142g (approximate thickness 6 mm), is positioned in the center of the Plexiglas® plate between the two end weights with the longest sides of the frame aligned with the longest sides of the plate.

If the specimen is smaller than 70 x 200 mm, or if a large enough area without a fold is not present, or if an area of interest is close to the edges of the specimen and can't be analyzed with the Plexiglas and weights settings described above, then the X-Y dimensions of the Plexiglas® plate and the added metal weights may be adjusted to reach a nominal external pressure of 1.86 kPa (0.27 psi) while maintaining a minimum 30 x 40 mm field of view. At least 10 complete three-dimensional protrusions of the specimen should be captured in the field of view of 30 mm x 40 mm.

Position the projection head to be normal to the specimen surface (i.e. to the topsheet of the topsheet/acquisition layer laminate).

Adjust the distance between the specimen and the projection head for best focus.

In the Primos Optical Profiler instrument, turn on the button "Pattern" to make a red cross appear on the screen ross and a black cross appears on the specimen.

Adjust the focus control until the black cross is aligned with the red cross on the screen.

Adjust image brightness then capture a digitized image.

In the Primos Optical Profiler instrument, change the aperture on the lens through the hole in the side of the projector head and/or altering the camera "gain" setting on the screen.

When the illumination is optimum, the red circle at the bottom of the screen labeled "I.O." will turn green.

Click on the "Measure" button.

The topology of the upper surface of the topsheet/acquisition layer laminate specimen is measured through the Plexiglas plate over the entire field of view 30 mm x 40 mm. It is important to keep the specimen still stationary during this time in order to avoid blurring of the captured image. The image should be captured within the 30 seconds following the placement of the Plexiglas plate, metal weights and frame on top of the specimen.

After the image has been captured, the X-Y-Z coordinates of every pixel of the 40 mm x 30 mm field of view area are recorded. The X direction is the direction parallel to the longest edge of the rectangular field of view, the Y direction is the direction parallel to the shortest edge of the rectangular field of view. The Z direction is the direction perpendicular to the X-Y plane. The X-Y plane is horizontal while the Z direction is vertical, i.e. orthogonal to the X-Y plane.

These data are smoothed and filtered using a polynomial filter (n = 6), a median filter 11 pixels by 11 pixels, and a structure filter 81 pixels by 81 pixels. The polynomial filter (n=6) approximates the X-Y-Z coordinate surface with a polynomial of order 6 and returns the difference to the approximated polynomial. The median filter 11 pixels by 11 pixels divides the field of view (40 mm x 30 mm) in X-Y squares of 11 pixels by 11 pixels. The Z coordinate of the pixel located at the center of a given 11 pixels by 11 pixels square will be replaced by the mean Z value of all the pixels of this given square. The structure filter 81 pixels by 81 pixels, removes the waviness of the structure and translates all the Z peak values belonging to the bottom surface of the Plexiglas plate to a top X-Y plane.

A Reference Plane is then defined as the X-Y plane intercepting the surface topology profile of the entire field of view (i.e. 30 mm x 40mm), 100 microns below this top X-Y plane. In the Primos Optical Profiler instrument, to measure the Material Area of the Reference Plane (Z=-0.1mm), click on the button "Evaluate". Then, apply a pre-filtering routine including a polynomial filter (n=6), a median filter 11 by 11 and a structure filter (n=81) using the function "Filter". Save the image to a computer file with ".omc" extension.

The same above procedure is then executed on the topsheet/acquisition layer laminate with the garment-facing side upward (i.e. the acquisition layer of the topsheet/acquisition layer laminate being upward), the 40 mm x 30 mm field of view being located at the exact same X-Y position of the topsheet/acquisition layer laminate.

The Empty Area of the reference plane can be defined as the area of the Reference Plane that is above the surface profile. The Empty Areas having boundaries strictly located inside the field of view area (i.e. 30 mm x 40 mm) without crossing or overlapping with the boundaries of the field of view area (i.e. 40 mm x 30 mm) are defined as Isolated Empty Area(s). The Measured Protrusion Base Width is defined for an Isolated Empty Area as the diameter of the biggest circle that can be inscribed inside a given Isolated Empty Area. This circle should only overlap with the Isolated Empty Area.

In the Primos Optical Profiler instrument, this can be done by clicking on "Draw circle" and drawing the biggest inscribed circle possible in a chosen Isolated Empty Area. Click on "Show sectional picture", the circle diameter can be measure via clicking on the extremity of the sectional picture profile and then clicking on "Horizontal distance" to obtain the Protrusion Base Width.

For both of the acquired and digitized images, the Protrusion Base Width of all the Isolated Empty Areas is determined. Then, the Measured Protrusion Base Width is calculated as the arithmetic average of the 6 biggest Protrusion Base Widths.

3) Protrusion Height Test Method

The topsheet/acquisition layer laminate is extracted from the absorbent article as described above in the Protrusion Base Width Test Method.

The topsheet/acquisition layer laminate specimen comprising three-dimensional protrusions is then conditioned and scanned under a pressure of 1.86 kPa (0.27 psi) with the body-facing side upward, i.e. the topsheet of the topsheet/acquisition layer laminate being upward as described above in the Protrusion Base Width Test Method.

After the image has been captured, the X-Y-Z coordinates of every pixel of the 40 mm x 30 mm field of view area are recorded and smoothed/filtered as described above in the Protrusion Base Width Test Method. A reference plane is also defined as described above in the Protrusion Base Width Test Method.

The Empty Area of the reference plane can be defined as the area of the Reference Plane that is above the surface profile. The Empty Area having boundaries strictly located inside the field of view area (i.e. 30 mm x 40 mm) without crossing or overlapping with the boundaries of the field of view area (i.e. 40 mm x 30 mm) are defined as Isolated Empty Area(s). The Protrusion Height is defined for an Isolated Empty Area as the distance between the minimum Z value of the points of the topsheet/acquisition layer laminate surface profile having X-Y coordinates located in this Isolated Empty Area, and the Z value of the top X-Y plane.

Click on "Draw N parallel lines" and draw a first segment parallel to the X axis of the field of view (direction of the longest dimension of the field of view) passing through the center of the Isolated Empty Area and extending outside the Isolated Empty Area boundaries. The center of the Isolated Empty Area corresponds to the middle of the segment parallel to the Y axis of the field of view and joining the biggest and smallest Y value of the Isolated Empty Area. Then input the "number" of lines to be drawn and set the "distance" between lines to 0.05 mm. Enough lines need to be drawn such to cover the entire Isolated Empty Area. Leave the averaging parameter to 0 then click "Ok". Then click on "Show sectional picture". Click on the point of the sectional picture profile having the minimum Z value and click on "Vertical distance" to obtain the Protrusion Height.

For both of the acquired and digitized images, the Protrusion Height of all the Isolated Empty Areas is determined. Then, the Measured Protrusion Height is calculated as the arithmetic average of the 6 biggest Protrusion Heights.

**Loss in Protrusion Height [%] after compression** is calculated as:

$$100 \ - \ \frac{\text{Protrusion Height at least 24 h after weight is removed x 100}}{\text{Protrusion Height before compression}}$$

As it has been found that the Protrusion Height does not significantly change further after 24 hours after the compression weight has been removed, the Protrusion height may be measured 24 hours after the compression weight has been removed, or, alternatively, more than 24 hours after the compression weight has been removed. For the data shown in Table 1, the Protrusion Height has been measured more than 24 hours after the weight has been removed.

## Accelerated Compression Method

1. Cut 10 samples of the topsheet/acquisition layer laminate 245 (called herein specimen) to be tested and 11 samples of paper towel into a 3 inch x 3 inch (7.6 cm x 7.6 cm) square.

2. Measure the caliper of each of the 10 specimens at 2.1 kPa and a dwell time of 2 seconds using a Thwing-Albert ProGage Thickness Tester or equivalent with a 50-60 millimeter diameter circular foot. Record the pre-compression caliper to the nearest 0.01 mm.

3. Alternate the layers of the specimens to be tested with the paper towels, starting and ending with the paper towels. The choice of paper towel does not matter and is present to prevent "nesting" of the protrusions in the deformed samples. The samples should be oriented so the edges of each of the specimens and each of the paper towels are relatively aligned, and the protrusions in the specimens are all oriented the same direction.

4. Place the stack of samples into a 40°C oven and place a weight on top of the stack. The weight must be larger than the foot of the thickness tester. To simulate high pressures or low in-bag stack heights, apply 35 kPa (e.g. 17.5 kg weight over a 70x70 mm area). To simulate low pressures or high in-bag stack heights, apply 7 kPa (e.g. 3.5 kg weight over a 70x70 mm area).

5. Leave the samples in the oven for 15 hours. After the time period has elapsed, remove the weight from the samples and remove the samples from the oven.

6. Within 30 minutes of removing the samples from the oven, measure the post-compression caliper as directed in step 2 above, making sure to maintain the same order in which the pre-compression caliper was recorded. Record the post-compression caliper of each of the 10 specimens to the nearest 0.01 mm.

7. Let the samples rest at 23 ± 2°C and at 50 ± 2% relative humidity for 24 hours without any weight on them.

8. After 24 hours, measure the post-recovery caliper of each of the 10 specimens as directed in step 2 above, making sure to maintain the same order in which the pre-compression and post-compression calipers were recorded. Record the post-recovery caliper of each of the 10 specimens to the nearest 0.01 mm. Calculate the amount of caliper recovery by subtracting the post-compression caliper from the post-recovery caliper and record to the nearest 0.01 mm.

9. If desired, an average of the 10 specimens can be calculated for the pre-compression, post-compression and post-recovery calipers.

## Examples

Topsheet of Example A:

The topsheet of Example A is a spunbonded nonwoven web made of monocomponent PP (polypropylene) fibers. The nonwoven topsheet has a basis weight of 20 g/m$^2$. The nonwoven topsheet is coated with a finish made of a fatty acid polyethylene glycol ester to render permanently hydrophilic. Coating is done prior to combining the topsheet with the acquisition layer to form the topsheet/acquisition layer laminate.

Topsheet of Comparative Examples A, B, C and D:

The topsheet of comparative Examples A, B, C and D is a spunbonded nonwoven web made of bi-component fibers. The fibers comprise a PP (polypropylene) core and PE (polyethylene) sheath. The nonwoven topsheet has a basis weight of 20 g/m$^2$. The nonwoven topsheet is coated with a finish made of a fatty acid polyethylene glycol ester to render permanently hydrophilic. Coating is done prior to combining the topsheet with the acquisition layer to form the topsheet/acquisition layer laminate.

Acquisition layer of Example A:

The following fibrous web was used as acquisition layer: The fibrous web has a basis weight of 70 g/m$^2$ and consists of 70% by weight of staple fibers (49 g/m$^2$) and 30% of latex binder (21 g/m$^2$). The staple fibers are a mixture of 6 denier solid round PE/PET concentric sheath/core bicomponent fibers (i.e. sheath made of polyethylene and core made of polyethylene terephthalate) and 1.2 denier solid trilobal PET monocomponent fibers. The staple fiber mixture consists of 70%, by total weight of the staple fibers, of bicomponent fibers and 30%, by total weight of the staple fibers, of trilobal monocomponent fibers.

The bicomponent fibers are commercially available from FiberVisions Corp. under the name ETC267CG3. The monocomponent fibers are commercially available from Kilop USA Inc. under the name Tarilin Nan Ya Y01. The latex binder is supplied by Omnova Solutions Inc. under the name Genflow 3160.

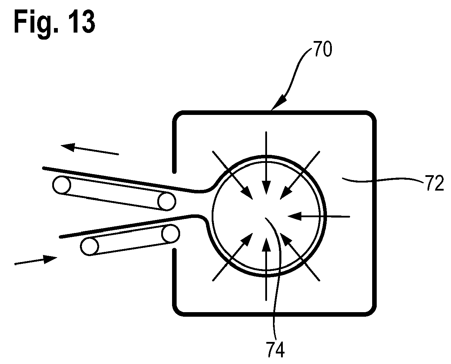

The mixture of staple fibers was laid down and underwent an air-through bonding step. An air-through bonder was used where the fibrous layer passes over a drum inside a hood, as exemplary shown in Fig. 13). The temperature in the bonder was set at 130°C. The fibrous layer traveled at a speed of 14.3 m/min with a residual time in the bonder of about 35 seconds. Subsequently, the latex binder was homogeneously applied onto the web by passing the web through a bath which contained the liquid latex binder (aqueous suspension with 20% binder) and the web was then subjected to curing by passing the web over drying cans at a temperature of about 150°C to 170°C and thereafter passing the web through an oven at 180°C to obtain the final web. Residual time in the oven was about 40 seconds. The web traveled at a speed of 13.9 m/min.

Acquisition layer of Comparative Example A:

The following fibrous web was used as acquisition layer: The fibrous web has a basis weight of 60 g/m$^2$ and consists of 100% staple fibers. The staple fibers are 6 denier solid round PE/PET concentric sheath/core bicomponent fibers (i.e. sheath made of polyethylene and core made of polyethylene terephthalate).

The bicomponent fibers are commercially available from FiberVisions Corp. under the name ETC267CG3.

The mixture of staple fibers was laid down and underwent an air-through bonding step. An air-through bonder was used where the fibrous layer passes over a drum inside a hood, as exemplary shown in Fig. 13). The temperature in the bonder was set at 130°C. The fibrous layer traveled at a speed of 14.6 m/min with a residual time in the bonder of about 35 seconds.

Acquisition layer of Comparative Example B:

The following fibrous web was used as acquisition layer: The fibrous web has a basis weight of 60 g/m$^2$ and consists of 100% staple fibers. The staple fibers are a mixture of 70% by weight of 6 denier solid round PE/PET concentric sheath/core bicomponent fibers (i.e. sheath made of polyethylene and core made of polyethylene terephthalate) and 30% by weight of 1.2 den trilobal PET.

The bicomponent fibers are commercially available from FiberVisions Corp. under the name ETC267CG3. The monocomponent fibers are commercially available from Kilop USA Inc. under the name Tarilin Nan Ya Y01.

The mixture of staple fibers was laid down and underwent an air-through bonding step. An air-through bonder was used where the fibrous layer passes over a drum inside a hood, as exemplary shown in Fig. 13). The temperature in the bonder was set at 133°C. The fibrous layer traveled at a speed of 18.8 m/min with a residual time in the bonder of about 30 seconds.

Acquisition layer of Comparative Example C:

The following fibrous web was used as acquisition layer: The fibrous web has a basis weight of 60 g/m$^2$ and

consists of 70% by weight of staple fibers (42 g/m$^2$) and 30% of latex binder (18 g/m$^2$). The staple fibers are a mixture of 6 denier solid round PET monocomponent fibers and 9 denier hollow round PET monocomponent fibers. The mixture consists of 50% (21 g/m$^2$), by total weight of the staple fibers, of the 6 denier monocomponent fibers and 50% (21 g/m$^2$), by total weight of the staple fibers, of the 9 denier monocomponent fibers.

The 6 denier monocomponent fibers are commercially available from Wellman Internat. Ltd. under the name H1327 Fillwell (hydrophilic). The 9 denier monocomponent fibers are commercially available from SILON s.r.o., Czech Republic.

The mixture of staple fibers was laid down and the latex binder was homogeneously applied onto the layer of fibers (i.e. the fibers are not subjected to any through air bonding and are thus not autogenously bonded). The acquisition layer is the same as the acquisition layer applied in commercially available Pampers Baby Dry® diapers in Germany in February 2016 (notably, the acquisition layer in the commercially available Pampers Baby Dry® diapers is not combined with the topsheet to form a topsheet/acquisition layer laminate).

Acquisition layer of Comparative Example D:

The following fibrous web was used as acquisition layer: The fibrous web has a basis weight of 60 g/m$^2$ and consists of 70% by weight of staple fibers (42 g/m$^2$) and 30% of latex binder (18 g/m$^2$). The staple fibers are a mixture of 4 denier solid round PET monocomponent fibers and 1.2 denier solid trilobal PET monocomponent fibers. The mixture consists of 50% (21 g/m$^2$), by total weight of the staple fibers, of the 4 denier monocomponent fibers and 50% (21 g/m$^2$), by total weight of the staple fibers, of the 1.2 denier trilobal monocomponent fibers.

The 4 denier monocomponent fibers are commercially available from Wellman Internat. Ltd. under the name T0888. The 1.2 denier monocomponent fibers are commercially available from Kilop USA Inc. under the name Tarilin Nan Ya Y01. The latex binder is supplied by Omnova Solutions Inc. under the name Genflow 3160.

The mixture of staple fibers was laid down and the latex binder was homogeneously applied onto the web by passing the web through a bath which contained the liquid latex binder (aqueous suspension with 40% binder) and the web was then subjected to curing by passing the web over drying cans at a temperature of about 150°C to 170°C and thereafter passing the web through an oven at 180°C to obtain the final web. Residual time in the oven was about 40 seconds. The web traveled at a speed of 13.3 m/min.

Formation of the topsheet/acquisition layer laminate in Example A and Comparative Examples A-D:

The topsheet and acquisition layer have been attached to each other with a hot melt adhesive applied in form of spirals with a basis weight of 2.2 g/m$^2$. Subsequently, the following steps were carried out:

The topsheet and acquisition layer attached together were simultaneously mechanically deformed by passing them between a pair of intermeshing male and female rolls. The protrusions were created such that the bases of the protrusions were present on the topsheet side (i.e. protrusions oriented towards the garment). The teeth on the male roll had a rounded diamond shape like that shown in Fig. 10A and Fig 10B, with vertical sidewalls. The teeth were 3.38 mm long and 2.77 mm wide with a CD spacing of 5.08 mm and an MD spacing of 8.79 mm. The recesses in the mating female roll also had a rounded diamond shape, similar to that of the male roll, with a clearance between the rolls of 0.53-1.09 mm. The process speed was 200 diapers per minutes and depth of engagement (DOE) was 3.43 mm, with the topsheet being in contact with the male roll and the AQL being in contact with the female roll.

Results (Table 1)

| Example | Protrusion Height (mm) | % Loss in Protrusion Height after 4kPa compression | Caliper: % recovery after 4kPa compression |
|---|---|---|---|
| Example A | 1.49 (before compression) | 17 | 75 |
| | 1.23 (at least 24 h after release of compression) | | |
| Comparative Example A | 1.04 (before compression) | 26 | 70 |
| | 0.77 (at least 24 h after release of compression) | | |

(continued)

| Example | Protrusion Height (mm) | % Loss in Protrusion Height after 4kPa compression | Caliper: % recovery after 4kPa compression |
|---|---|---|---|
| Comparative Example B | 0.64 (before compression) | 34 | 68 |
| | 0.42 (at least 24 h after release of compression) | | |
| Comparative Example C | 0.80 (before compression) | 46 | 74 |
| | 0.43 (at least 24 h after release of compression) | | |
| Comparative Example D | 0.47 (before compression) | 28 | 75 |
| | 0.34 (at least 24 h after release of compression) | | |

Example A, prior to compression, provides a significantly higher Protrusion Height than all Comparative Examples. After compression Example A has significantly reduced loss in Protrusion Height compared to all Comparative Examples.

**Examples of carded air-through bonded nonwoven fibrous web with latex binder (prior to being combined with topsheet to form a laminate)**

Example 1: Through air bonded nonwoven fibrous web with latex binder.

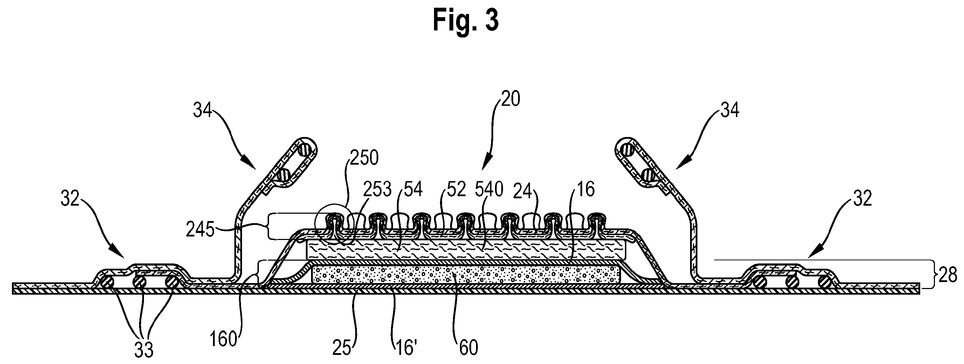

The fibrous web has a basis weight of 60 g/m$^2$ and consists of 80% by weight of staple fibers (48 g/m$^2$) and 20% of latex binder (12 g/m$^2$). The staple fibers are a mixture of 6 denier solid round PE/PET concentric sheath/core bicomponent fibers (i.e. sheath made of polyethylene and core made of polyethylene terephthalate) and 1.2 denier solid trilobal PET monocomponent fibers. The mixture consists of 80%, by total weight of the staple fibers, of bicomponent fibers and 20%, by total weight of the staple fibers, of trilobal monocomponent fibers.
The bicomponent fibers are commercially available from FiberVisions Corp. under the name ETC267CG3. The monocomponent fibers are commercially available from Kilop USA Inc. under the name Tarilin Nan Ya Y01. The latex binder is supplied by Omnova Solutions Inc. under the name Genflow 3160.
The mixture of staple fibers was laid down and underwent an air-through bonding step. An air-through bonder was used where the fibrous layer passes over a drum inside a hood, as exemplary shown in Fig. 3). The temperature in the bonder was set at 130°C. The fibrous layer traveled at a speed of 14.3 m/min with a residual time in the bonder of about 35 seconds. Subsequently, the latex binder was homogeneously applied onto the web by passing the web through a bath which contained the liquid latex binder (aqueous suspension with 20% binder) and the web was then subjected to curing by passing the web over drying cans at a temperature of about 150°C to 170°C and thereafter passing the web through an oven at 180°C to obtain the final web. Residual time in the oven was about 40 seconds. The web traveled at a speed of 13.9 m/min.

Comparative Example 1: Through air bonded nonwoven fibrous web without latex binder.

The fibrous web consists of 100% by weight of staple fibers (60 g/m$^2$). The staple fibers are a mixture of 6 denier solid round PE/PET concentric sheath/core bicomponent fibers (i.e. sheath made of polyethylene and core made of polyethylene terephthalate) and 1.2 denier solid trilobal PET monocomponent fibers. The mixture consists of 80%, by total weight of the staple fibers, of bicomponent fibers and 20%, by total weight of the staple fibers, of trilobal monocomponent fibers.
The bicomponent fibers are commercially available from FiberVisions Corp. under the name ETC267CG3. The monocomponent fibers are commercially available from Kilop USA Inc. under the name Tarilin Nan Ya Y01.
The mixture of staple fibers was laid down and underwent an air-through bonding step. An air-through bonder was used where the fibrous layer passes over a drum inside a hood, as exemplary shown in Fig. 3). The temperature in the bonder was set at 130°C. The fibrous layer traveled at a speed of 14.3 m/min with a residual time in the bonder of about 35 seconds.

Example 2: Through air bonded nonwoven fibrous web with latex binder.

The fibrous web consists of 80% by weight of staple fibers (48 g/m$^2$) and 20% of latex binder (12 g/m$^2$). The staple fibers are a mixture of 6 denier solid round CoPET/PET concentric sheath/core bicomponent fibers (i.e. sheath made of CoPET and core made of polyethylene terephthalate) and 1.2 denier solid trilobal PET monocomponent fibers. The mixture consists of 80%, by total weight of the staple fibers, of bicomponent fibers and 20%, by total weight of the staple fibers, of trilobal monocomponent fibers.

The bicomponent fibers are commercially available from Toray Chemical Int. under the name Eslom UL007. The monocomponent fibers are commercially available from Kilop USA Inc. under the name Tarilin Nan Ya Y01. The latex binder is supplied by Omnova Solutions Inc. under the Genflow 3160.

The mixture of staple fibers was laid down and underwent an air-through bonding step. An air-through bonder was used where the fibrous layer passes over a drum inside a hood, as exemplary shown in Fig. 3). The temperature in the bonder was set at 150°C. The fibrous layer traveled at a speed of 14.2 m/min with a residual time in the bonder of about 35 seconds. Subsequently, the latex binder was homogeneously applied onto the web by passing the web through a bath which contained the liquid latex binder (aqueous suspension with 20% binder) and the web was then subjected to curing by passing the web over drying cans at a temperature of about 150°C to 170°C and thereafter passing the web through an oven at 180°C to obtain the final web. Residual time in the oven was about 40 seconds. The web traveled at a speed of 13.9 m/min.

Comparative Example 2: Through air bonded nonwoven fibrous web with latex binder.

The fibrous web consists of 100% by weight of staple fibers (60 g/m$^2$). The staple fibers are a mixture of 6 denier solid round CoPET/PET concentric sheath/core bicomponent fibers (i.e. sheath made of CoPET and core made of polyethylene terephthalate) and 1.2 denier solid trilobal PET monocomponent fibers. The mixture consists of 80%, by total weight of the staple fibers, of bicomponent fibers and 20%, by total weight of the staple fibers, of trilobal monocomponent fibers.

The bicomponent fibers are commercially available from Toray Chemical Int. under the name Eslom UL007. The monocomponent fibers are commercially available from Kilop USA Inc. under the name Tarilin Nan Ya Y01.

The mixture of staple fibers was laid down and underwent an air-through bonding step. An air-through bonder was used where the fibrous layer passes over a drum inside a hood, as exemplary shown in Fig. 3). The temperature in the bonder was set at 150°C. The fibrous layer traveled at a speed of 14.2 m/min with a residual time in the bonder of about 35 seconds.

Results (Table 2)

| | Example 1 | Comparative Example 1 | Example 2 | Comparative Example 2 |
|---|---|---|---|---|
| Caliper Recovery after Compression at 12 kPa [%] | 68.3 | 63.6 | 58.4 | 53.0 |
| Caliper Recovery after Compression at 4 kPa [%] | 84.5 | 82.4 | 79.7 | 69.4 |
| Caliper Recovery after Compression at 1 kPa [%] | 96.9 | 93.5 | 96.7 | 87.6 |
| Air Permeability before compression [m$^3$/m$^2$/min] | 333 | 246 | 315 | 303 |
| IPRP before compression [10$^6$ (cm/Pa sec)] | 16,874 | 12,696 | 21,208 | 19,293 |
| IPRP 24h after compression at 4 kPa [10$^6$ (cm/Pa sec)] | 13,384 | 10,180 | 21,368 | 16,783 |

The data show that by subjected to air-through bonded carded nonwoven fibrous web to subsequent treatment with a latex binder and curing the web, the Caliper Recovery after compression at 12 kPa improves by 7% for Example 1 vs. Comparative Example 1, and by 10% for Example 2 vs. Comparative Example 2.

Caliper Recovery after compression at 4 kPa improves by 2.5% for Example 1 vs. Comparative Example 1, and by 15% for Example 1 vs. Comparative Example 1.

Caliper Recovery after compression at 1 kPa improves by 4% for Example 1 vs. Comparative Example 1, and by 10% for Example 2 vs. Comparative Example 2.

Also Air Permeability (measured without having the carded nonwoven fibrous web subjected to compression) and In Plane Radial Permeability (IPRP) (measured without having the carded nonwoven fibrous webs subjected to compression prior to testing as well as measured for the webs 24 h after they were compressed at 4 kPa) is improved with additional treatment with a latex binder:

Air Permeability increases by 35% for Example 1 vs. Comparative Example 1, and by 4% for Example 2 vs. Comparative Example 2.

IPRP increases by 33% for Example 1 vs. Comparative Example 1, and by 10% for Example 2 vs. Comparative Example 2 for the webs without previous compression. IPRP increases by 31% for Example 1 vs. Comparative Example 1, and by 27% for Example 2 vs. Comparative Example 2 for the webs 24 h after they were compressed at 4 kPa.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

**Claims**

1. An absorbent article for personal hygiene comprising:

   a longitudinal axis, a transversal axis perpendicular to the longitudinal axis, a liquid permeable topsheet, an acquisition layer, a liquid impermeable backsheet and an absorbent core, wherein the absorbent core is located between the topsheet and backsheet;
   wherein the topsheet and acquisition layer are jointed to each other to form a topsheet/acquisition layer laminate comprising the liquid permeable topsheet and the acquisition layer in a face to face relationship, wherein the topsheet/acquisition layer laminate comprises three-dimensional protrusions extending from a plane of the topsheet/acquisition layer laminate;
   wherein a width of the acquisition layer in a direction parallel to the transversal axis is less than a width of the topsheet in a direction parallel to the transversal axis; and
   wherein the acquisition layer comprises a carded nonwoven fibrous web comprising at least 50%, by weight of the fibrous web, of staple fibers and at least 10%, by weight of the fibrous web, of non-fibrous latex binder, wherein, the staple fibers are autogenously bonded to each other and are bonded to each other by the latex binder.

2. The absorbent article of claim 1, wherein the latex binder exhibits a larger degree of delamination from the staple fibers in the three-dimensional protrusions than in the plane areas of the topsheet/acquisition layer laminate.

3. The absorbent article of claim 1 or 2, wherein the topsheet/acquisition layer laminate has a loss in Protrusion Height of less than 25% after compression with an applied pressure of 4 kPa according to the test method set out herein.

4. The absorbent article of any of the preceding claims, wherein the topsheet/acquisition layer laminate has a caliper recovery of at least 70% after compression with an applied pressure of 4 kPa according to the test method set out herein.

5. The absorbent article of any of the preceding claims, wherein the carded nonwoven fibrous web comprises a mixture of at least a first and a second type of staple fibers.

6. The absorbent article of claim 5, wherein the second type of fibers are shaped fibers.

7. The absorbent article of any of claims 5 or 6, wherein the staple fibers of the carded nonwoven fibrous web comprise at least 20%, by total weight of the staple fibers, of the first type of fibers.

8. The absorbent article of any of claims 5 to 7, wherein the staple fibers of the carded nonwoven fibrous web comprise at least 5%, by total weight of the staple fibers, of the second type of fibers.

9. The absorbent article of any of claims 5 to 8, wherein the carded nonwoven fibrous web comprises from 65 to 95%, by total weight of the staple fibers, of the first type of fibers and from 5% to 35%, by total weight of the staple fibers, of the second type of fibers.

10. The absorbent article of any of the preceding claims, wherein autogenous bonding of the staple fibers of the carded nonwoven fibrous web was carried out prior to application of the binder.

11. The absorbent article of any of the preceding claims, wherein the staple fibers of the carded nonwoven fibrous web are made of polypropylene, polyethylene, polyester, polyethylene terephthalate (PET), CoPET, polybutylene terephthalate, polyamide, polylactic acid, viscose, and combinations thereof.

12. The absorbent article of any of the preceding claims, wherein the latex binder of the carded nonwoven fibrous web is a styrene-butadiene copolymer binder.

13. The absorbent article of any of the preceding claims, wherein the three-dimensional protrusions of the topsheet/acquisition layer laminate are formed from the fibers of the topsheet and the acquisition layer, wherein a majority of the three-dimensional protrusions each comprise a base forming an opening, an opposed distal portion and one or more side walls between the base and the distal portion, wherein the base, the distal portion, and the one or more side walls are formed by fibers such that the majority of the three-dimensional protrusions have openings at the base.

14. The absorbent article of claim 13, wherein the topsheet and the acquisition layer of the topsheet/acquisition layer laminate are nested together, such that a majority of the three-dimensional protrusions of the topsheet and of the acquisition layer coincide such that the protrusions of the topsheet fit into the protrusions of the acquisition layer.

15. The absorbent article according to any of the preceding claims wherein the liquid permeable topsheet and the acquisition layer are in intimate contact with each other.

**Fig. 1**

# Fig. 2

EP 3 216 435 A1

Fig. 3

28

32

34

16

20

52 540 24

253 54

250

245

160

33

32

34

60

16'

25

# Fig. 4

245  12  24

42  250

60

25

160 { 16'
     16

540

28

54

52

5

32

34

13

20  44  10  80  90

38

37

36

5

**Fig. 5**

EP 3 216 435 A1

# Fig. 6

Fig. 7

EP 3 216 435 A1

# Fig. 8

# Fig. 9

# Fig. 10A

Fig. 10B

**Fig. 10C**

## Fig. 11A

## Fig. 11C

## Fig. 11B

EP 3 216 435 A1

Fig. 11D

Fig. 11E

Fig. 11F

Fig. 12A

Fig. 12B

Fig. 12C

Fig. 12D

Fig. 12E

# Fig. 13

Fig. 14

Fig. 15

**Fig. 16**

⊢⊣ 200 µm

## Fig. 17

⊢—⊣ 200 µm

# Fig. 18

⊢⊣ 200 µm

## Fig. 19

**Europäisches Patentamt**
**European Patent Office**
**Office européen des brevets**

# EUROPEAN SEARCH REPORT

Application Number

EP 16 15 9461

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2015/173968 A1 (JOSEPH LAVEETA [US]) 25 June 2015 (2015-06-25) * paragraph [0101] - paragraph [0110]; figures 2,3 * | 1-15 | INV. A61F13/511 A61F13/537 A61F13/512 |
| Y | US 2015/065973 A1 (ROE DONALD CARROLL [US] ET AL) 5 March 2015 (2015-03-05) * paragraph [0103] - paragraph [0109]; figures 2,3 * | 1-15 | |
| Y | US 2009/270825 A1 (WCIORKA MAJA [DE] ET AL) 29 October 2009 (2009-10-29) * paragraph [0089] - paragraph [0090] * | 1-15 | |
| Y | US 2012/238982 A1 (WEISMAN PAUL THOMAS [US] ET AL) 20 September 2012 (2012-09-20) * paragraphs [0205], [0265]; figures 1-18 * | 1-15 | |
| Y | US 5 876 388 A (MCDOWALL DEBRA JEAN [US] ET AL) 2 March 1999 (1999-03-02) * column 4, line 65 - column 5, line 40; figure 1 * | 1-15 | TECHNICAL FIELDS SEARCHED (IPC) A61F |

The present search report has been drawn up for all claims

| Place of search | Date of completion of the search | Examiner |
|---|---|---|
| Munich | 8 November 2016 | Gennari, Silvia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 15 9461

This annex lists the patent family members relating to the patent documents cited in the above-mentioned European search report.
The members are as contained in the European Patent Office EDP file on
The European Patent Office is in no way liable for these particulars which are merely given for the purpose of information.

08-11-2016

| Patent document cited in search report | | Publication date | Patent family member(s) | | Publication date |
|---|---|---|---|---|---|
| US 2015173968 | A1 | 25-06-2015 | CA | 2933890 A1 | 25-06-2015 |
| | | | CN | 104720984 A | 24-06-2015 |
| | | | CN | 204766177 U | 18-11-2015 |
| | | | EP | 3082699 A2 | 26-10-2016 |
| | | | KR | 20160087860 A | 22-07-2016 |
| | | | US | 2015173968 A1 | 25-06-2015 |
| | | | WO | 2015095514 A2 | 25-06-2015 |
| US 2015065973 | A1 | 05-03-2015 | CA | 2922316 A1 | 05-03-2015 |
| | | | CN | 105473113 A | 06-04-2016 |
| | | | EP | 3038578 A1 | 06-07-2016 |
| | | | JP | 2016530961 A | 06-10-2016 |
| | | | US | 2015065973 A1 | 05-03-2015 |
| | | | US | 2015065976 A1 | 05-03-2015 |
| | | | WO | 2015031229 A1 | 05-03-2015 |
| US 2009270825 | A1 | 29-10-2009 | AR | 071219 A1 | 02-06-2010 |
| | | | CA | 2721974 A1 | 05-11-2009 |
| | | | CN | 102014824 A | 13-04-2011 |
| | | | EP | 2273959 A1 | 19-01-2011 |
| | | | JP | 2011518613 A | 30-06-2011 |
| | | | US | 2009270825 A1 | 29-10-2009 |
| | | | US | 2015230999 A1 | 20-08-2015 |
| | | | WO | 2009134626 A1 | 05-11-2009 |
| US 2012238982 | A1 | 20-09-2012 | BR | 112013021967 A2 | 20-09-2016 |
| | | | CA | 2830211 A1 | 20-09-2012 |
| | | | CN | 103429277 A | 04-12-2013 |
| | | | EP | 2686024 A1 | 22-01-2014 |
| | | | JP | 5788536 B2 | 30-09-2015 |
| | | | JP | 2014507252 A | 27-03-2014 |
| | | | US | 2012238982 A1 | 20-09-2012 |
| | | | WO | 2012125538 A1 | 20-09-2012 |
| US 5876388 | A | 02-03-1999 | AU | 695130 B2 | 06-08-1998 |
| | | | AU | 1619295 A | 12-10-1995 |
| | | | BR | 9501235 A | 07-11-1995 |
| | | | CA | 2129210 A1 | 01-10-1995 |
| | | | DE | 69528484 D1 | 14-11-2002 |
| | | | DE | 69528484 T2 | 13-02-2003 |
| | | | EP | 0674891 A2 | 04-10-1995 |
| | | | JP | H07275293 A | 24-10-1995 |
| | | | KR | 100361784 B1 | 29-01-2003 |
| | | | TW | 305892 B | 21-05-1997 |
| | | | US | 5700254 A | 23-12-1997 |
| | | | US | 5876388 A | 02-03-1999 |

EPO FORM P0459

For more details about this annex : see Official Journal of the European Patent Office, No. 12/82

page 1 of 2

**ANNEX TO THE EUROPEAN SEARCH REPORT
ON EUROPEAN PATENT APPLICATION NO.**                    EP 16 15 9461

This annex lists the patent family members relating to the patent documents cited in  the above-mentioned European search report.
The members are as contained in the European Patent Office EDP file on
The European Patent Office is in no way liable for these particulars which are merely given for the purpose of information.

08-11-2016

| Patent document cited in search report | Publication date | Patent family member(s) | Publication date |
|---|---|---|---|
| | | ZA      9501878  B | 11-12-1995 |

For more details about this annex : see Official Journal of the European Patent Office, No. 12/82

EPO FORM P0459

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3860003 A
- US 5221274 A
- US 5554145 A
- US 5569234 A
- US 5580411 A
- US 6004306 A
- US 5151092 A, Buell
- US 5599335 A, Goldman
- EP 1447066 A1, Busam

- WO 9511652 A, Tanzer
- US 20080312622 A1, Hundorf
- WO 2012052172 A, Van Malderen
- US 7744576 B
- US 20110268932 A1
- US 20110319848 A1
- US 20110250413 A1
- US 5166259 A, Schmeing and White

**Non-patent literature cited in the description**

- **SCHWARTZ ; BERRY.** Surface Active Agents. Interscience Publishers, Inc, 1958, vol. 1
- **MOILET ; COLLIE ; BLACK, D.** Surface Activity. Van Nostrand Company, Inc, 1961

- **FEISER ; FEISER, D.C.** Organic Chemistry. Heath and Company, 1944
- The Merck Index. Merck & Co., Inc, 1960
- **J.D. LINDSAY.** The anisotropic Permeability of Paper. *TAPPI Journal,* May 1990, 223